# EUROPÄISCHE PATENTSCHRIFT

(11) **EP 2 132 248 B1**
(45) Veröffentlichungstag und Bekanntmachung des Hinweises auf die Patenterteilung: **13.07.2016**
(21) Anmeldenummer: 08735553.3
(22) Anmeldetag: 28.03.2008
(51) Int. Cl.: C08G 18/32, C07C 251/06, C08G 18/10

(54) **ALDIMINE MIT AKTIVEM WASSERSTOFF AUFWEISENDEN REAKTIVGRUPPEN**
ALDIMINES WITH REACTIVE GROUPS COMPRISING ACTIVE HYDROGEN
ALDIMINES COMPORTANT DES GROUPEMENTS RÉACTIFS MUNIS D'HYDROGÈNE ACTIF

(30) Priorität: 28.03.2007 EP 07105146
(43) Veröffentlichungstag der Anmeldung: 16.12.2009
(73) Patentinhaber: Sika Technology AG, 6340 Baar (CH)
(72) Erfinder: BURCKHARDT, Urs, CH-8049 Zürich (CH)
(74) Vertreter: Sika Patent Attorneys
(86) Internationale Anmeldenummer: PCT/EP2008/053712
(87) Internationale Veröffentlichungsnummer: WO 2008/116928

(56) Entgegenhaltungen:
- EP-A- 1 384 735
- EP-A- 1 524 282
- EP-A- 1 770 107
- EP-A- 1 772 447
- EP-A- 1 775 284

## Beschreibung

### Technisches Gebiet

Die Erfindung betrifft das Gebiet der Aldimine, sowie das Gebiet der feuchtigkeitshärtenden Polyurethane.

### Stand der Technik

Aldimine sind Kondensationsprodukte aus primären Aminen und Aldehyden und stellen eine seit langem bekannte Stoffklasse dar. Bei Kontakt mit Wasser können Aldimine zu den entsprechenden Aminen und Aldehyden hydrolysieren, während sie in Abwesenheit von Wasser stabil sind. Aufgrund dieser Eigenart können sie als gebundene oder geschützte Form von Aminen, beziehungsweise Aldehyden, verwendet werden. So werden Aldimine beispielsweise in der Polyurethanchemie eingesetzt, wo sie als durch Feuchtigkeit aktivierbare Vernetzer, sogenannte "latente Amine" oder "latente Härter", für Isocyanatgruppen-haltige Polyurethanzusammensetzungen dienen. Die Verwendung eines Aldimins als latenten Härter in Isocyanatgruppen-haltigen Systemen hat dabei zweierlei Vorteile: zum einen lässt sich die Entstehung von unerwünschten Gasblasen im ausgehärteten Kunststoff vermeiden, da die Aushärtung über das latente Amin - im Gegensatz zur direkten Reaktion des Isocyanats mit Feuchtigkeit - nicht unter Freisetzung von Kohlendioxid (CO₂) verläuft; zum anderen lassen sich höhere Aushärtungsgeschwindigkeiten erzielen. Die Verwendung von Aldiminen in feuchtigkeitshärtenden Polyurethanen kann aber auch Probleme verursachen. So kann, je nach vorhandenen Isocyanat- und Aldiminogruppen, die Lagerstabilität einer Zusammensetzung stark eingeschränkt sein. Weiterhin weisen Aldiminogruppen-haltige Polyurethanzusammensetzungen - je nach den bei der Herstellung des Aldimins verwendeten Aldehyden - oft einen sehr starken Geruch auf, welcher für viele Anwendungen nicht tolerierbar ist.

Aldimine, welche zusätzliche funktionelle Gruppen aufweisen, sind bekannt. Sie haben die Eigenschaft, dass sie mit geeigneten reaktiven Verbindungen Addukte bilden, welche vielseitig verwendbar sind.

US 4,224,417 und US 6,136,942 offenbaren Aldimine mit gegenüber Isocyanaten reaktiven funktionellen Gruppen, wobei diese Aldimine ausgehend von primären oder sekundären aliphatischen Aldehyden oder aromatischen Aldehyden hergestellt sind. Zusammensetzungen enthaltend solche Aldimine weisen aber eine unzureichende Lagerstabilität auf und/oder zeigen in ausgehärteter Form Probleme mit der Lichtechtheit.

### Darstellung der Erfindung

Aufgabe der vorliegenden Erfindung ist es daher, Aldimine mit zusätzlichen funktionellen Gruppen zur Verfügung zu stellen, welche die Nachteile des Standes der Technik überwinden.

Überraschenderweise lässt sich diese Aufgabe mittels den in Anspruch 1 offenbarten neuen Aldiminen lösen. Diese Aldimine weisen neben mindestens einer Aldiminogruppe mindestens eine einen aktiven Wasserstoff enthaltende Gruppierung in der Form einer Hydroxyl-, Mercapto- oder sekundären Aminogruppe auf.

Die Aldimine sind lagerstabil und lassen sich auf einfache Art mit gegenüber aktivem Wasserstoff reaktiven Verbindungen zu Reaktionsprodukten umsetzen, insbesondere zu Aldiminogruppen-haltigen Verbindungen gemäss Anspruch 3. Derartige Reaktionsprodukte können unter dem Einfluss von Wasser zu Vernetzungsreaktionen Anlass geben. In einer bevorzugten Ausführungsform stellen die Reaktionsprodukte Verbindungen gemäss Anspruch 4 dar, welche neben mindestens einer Aldiminogruppe mindestens eine Isocyanatgruppe aufweisen. Diese Reaktionsprodukte sind äusserst lagerstabil, auch wenn Reaktivgruppen, wie insbesondere Isocyanatgruppen, darin vorhanden sind. Zudem weisen sie vor, während und nach der Hydrolyse nur einen geringen Geruch auf.

Ein weiterer Aspekt der Erfindung betrifft Zusammensetzungen gemäss Anspruch 7. Insbesondere können derartige Zusammensetzungen als Klebstoff, Dichtstoff, Vergussmasse oder Beschichtung gemäss Anspruch 11 verwendet werden. Insbesondere sind sie als Heissschmelzklebstoffe oder Primer geeignet. Mit Wasser reagieren derartige Zusammensetzungen zu ausgehärteten Zusammensetzungen gemäss Anspruch 10.

Weitere Aspekte der vorliegenden Erfindung betreffen ein Verfahren zum Verkleben gemäss Anspruch 12, ein Verfahren zum Abdichten gemäss Anspruch 13, ein Verfahren zum Beschichten gemäss Anspruch 14 sowie die daraus resultierenden Artikel gemäss Anspruch 15.

Es wurde überraschenderweise gefunden, dass mittels der Verwendung von Aldiminen gemäss Anspruch 1 der Gehalt an monomeren Diisocyanaten in Polyurethanpolymeren sowie in Polyurethanpolymeren enthaltenden Zusammensetzungen, stark reduziert werden kann.

Deshalb stellt ein Verfahren zur Verminderung des Gehaltes an monomeren Diisocyanaten gemäss Anspruch 16 einen besonderen Aspekt der vorliegenden Erfindung dar. Dieses Verfahren erlaubt es in eleganter Art und Weise, ein sehr grosses Problem der Polyurethanchemie, nämlich das Vorhandensein von Anteilen an unerwünschten Diisocyanat-Monomeren kostengünstig und effizient zu lösen, mit dem Vorteil auf, dass die zweifache Funktionalität der Monomere nicht verloren geht, so dass diese nutzbringend in den bei der Vernetzung der Polyurethanpolymere entstehenden hochmolekularen Kunststoff eingebaut werden.

Besonders bevorzugte Ausführungsformen der Erfindung sind Gegenstand der abhängigen Ansprüche.

### Wege zur Ausführung der Erfindung

Gegenstand der Erfindung sind Aldimine der Formel (I), 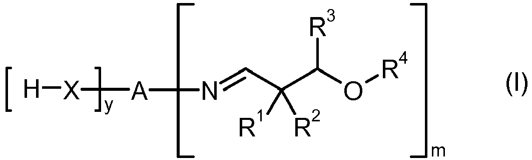 wobei
R¹ und R² entweder
   unabhängig voneinander jeweils für einen einwertigen Kohlenwasserstoffrest mit 1 bis 12 C-Atomen stehen,
   oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder für eine Alkyl- oder Arylalkylgruppe, insbesondere mit 1 bis 12 C-Atomen, steht;
R⁴ entweder
   für einen Kohlenwasserstoffrest mit 1 bis 30 C-Atomen, der gegebenenfalls Ethersauerstoffatome aufweist, steht,
   oder für einen Rest 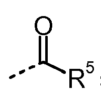 steht, wobei R⁵ für ein Wasserstoffatom oder für einen Kohlenwasserstoffrest mit 1 bis 5 C-Atomen steht;
A entweder
   für einen (m+y)-wertigen, gegebenenfalls Heteroatome aufweisenden, Kohlenwasserstoffrest mit 2 bis 20 C-Atomen steht,
   oder, falls y für 1 und X für N-R⁷ steht, zusammen mit R⁷ für einen (m+2)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
X für O oder S oder N-R⁶ oder N-R⁷ steht,
   wobei R⁶
      entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
      oder für einen Substituenten der Formel (II) steht, 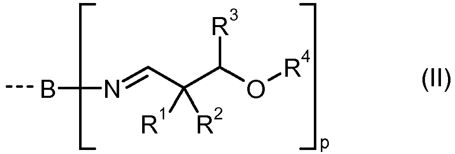 wobei
      p für 0 oder für eine ganze Zahl von 1 bis 10'000 steht, und
      B für einen (p+1)-wertigen Kohlenwasserstoffrest, welcher gegebenenfalls Heteroatome, insbesondere in Form von Ether-Sauerstoff oder tertiärem Amin-Stickstoff, und gegebenenfalls aktiven Wasserstoff in Form von Hydroxylgruppen, sekundären Aminogruppen oder Mercaptogruppen enthält, steht, und
   R⁷ zusammen mit A für einen (m+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht; und
m für 1 oder 2 oder 3 oder 4 steht, und
y für 1 oder 2 oder 3 oder 4 steht.

Die gestrichelten Linien in den Formeln in diesem Dokument stellen jeweils die Bindung zwischen einem Substituenten und dem zugehörigen Molekülrest dar.

Der Begriff "primäre Aminogruppe" bezeichnet im vorliegenden Dokument eine NH₂-Gruppe, die an einen organischen Rest gebunden ist, während der Begriff "sekundäre Aminogruppe" eine NH-Gruppe bezeichnet, die an zwei organische Reste, welche auch gemeinsam Teil eines Rings sein können, gebunden ist, und der Begriff "tertiäre Aminogruppe" oder "tertiärer Amin-Stickstoff" ein Stickstoffatom bezeichnet, das an drei organische Reste gebunden ist, wobei diese Reste auch über Ringe verbunden sein können.

Der Begriff "aktiver Wasserstoff' bezeichnet im vorliegenden Dokument ein deprotonierbares, an ein Stickstoff-, Sauerstoff- oder Schwefelatom gebundenes Wasserstoffatom.

In den Formeln (I) und (II) stehen R¹ und R² bevorzugt für je eine Methylgruppe. Weiterhin steht R³ bevorzugt für ein Wasserstoffatom; R⁴ steht bevorzugt für einen Rest 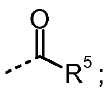 y steht bevorzugt für 1; (y+m) steht bevorzugt für 2 oder 3, insbesondere für 2.

Die Reste R¹, R², R³ und R⁴ der Formel (I) weisen keine Gruppierungen auf, die mit Isocyanatgruppen reaktionsfähig sind; insbesondere weisen R¹, R², R³ und R⁴ keine Hydroxylgruppen, keine primären oder sekundären Aminogruppen und keine Mercaptogruppen auf.

Die Aldimine der Formel (I) sind beispielsweise erhältlich aus der Umsetzung von mindestens einem Amin **B1** der Formel (III) mit mindestens einem Aldehyd **ALD** der Formel (IV), 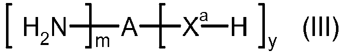 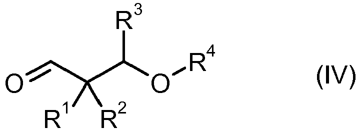 wobei
X^{a} für O oder S oder N-R^{6a} oder N-R⁷ steht,
   wobei R^{6a}
      entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
      oder für einen Substituenten der Formel (III a) steht, 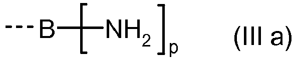
   und A, B, R¹, R², R³, R⁴, R⁷, m, y und p die bereits genannten Bedeutungen aufweisen.

Die Umsetzung zwischen dem Amin **B1** der Formel (III) und dem Aldehyd **ALD** der Formel (IV) erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **ALD** wird hierbei in Bezug auf die primären Aminogruppen des Amins **B1** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (I) wird jedoch ein Herstellverfahren ohne Verwendung von Lösemitteln bevorzugt, wobei das bei der Kondensation gebildete Wasser durch Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen.

Für die Umsetzung zum Aldimin der Formel (I) geeignete Aldehyde **ALD** der Formel (IV) sind in einer Ausführungsform Aldehyde **ALD1** der Formel (IV a), 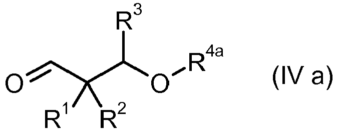 wobei R^{4a} für einen Kohlenwasserstoffrest mit 1 bis 30 C-Atomen, der gegebenenfalls Ethersauerstoffatome aufweist, steht.

Die Aldehyde **ALD1** der Formel (IV a) stellen Ether von aliphatischen, cycloaliphatischen oder arylaliphatischen 2,2-disubstituierten 3-Hydroxy-aldehyden mit Alkoholen oder Phenolen der Formel R^{4a}-OH dar, beispielsweise Fettalkoholen oder Phenol. Geeignete 2,2-disubstituierte 3-Hydroxy-aldehyde sind ihrerseits erhältlich aus Aldol-Reaktionen, insbesondere gekreuzten Aldol-Reaktionen, zwischen primären oder sekundären aliphatischen Aldehyden, insbesondere Formaldehyd, und sekundären aliphatischen, sekundären cycloaliphatischen oder sekundären arylaliphatischen Aldehyden, wie beispielsweise Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd (Hydratropaldehyd) oder Diphenylacetaldehyd. Beispiele geeigneter 2,2-disubstituierter 3-Hydroxyaldehyde sind 2,2-Dimethyl-3-hydroxypropanal, 2-Hydroxymethyl-2-methyl-butanal, 2-Hydroxymethyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethyl-cyclohex-3-encarboxaldehyd, 2-Hydroxymethyl-2-methyl-3-phenyl-propanal, 3-Hydroxy-2-methyl-2-phenyl-propanal und 3-Hydroxy-2,2-diphenyl-propanal.

Beispiele für geeignete Aldehyde **ALD1** der Formel (IV a) sind 2,2-Dimethyl-3-phenoxy-propanal, 3-Cyclohexyloxy-2,2-dimethyl-propanal, 2,2-Dimethyl-3-(2-ethylhexyloxy)-propanal, 2,2-Dimethyl-3-lauroxy-propanal und 2,2-Dimethyl-3-stearoxy-propanal.

Für die Umsetzung zum Aldimin der Formel (I) geeignete Aldehyde **ALD** der Formel (IV) sind in einer weiteren Ausführungsform Aldehyde **ALD2** der Formel (IV b), 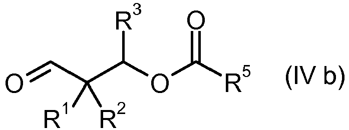 wobei R⁵ für ein Wasserstoffatom oder für einen Kohlenwasserstoffrest mit 1 bis 5 C-Atomen steht.

Die Aldehyde **ALD2** der Formel (IV b) stellen Ester der bereits beschriebenen 2,2-disubstituierten 3-Hydroxyaldehyde, wie beispielsweise 2,2-Dimethyl-3-hydroxypropanal, 2-Hydroxymethyl-2-methyl-butanal, 2-Hydroxy-methyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxy-methyl-cyclohexancarboxaldehyd 1-Hydroxymethyl-cyclohex-3-encarboxalde-hyd, 2-Hydroxymethyl-2-methyl-3-phenyl-propanal, 3-Hydroxy-2-methyl-2-phenyl-propanal und 3-Hydroxy-2,2-diphenyl-propanal, mit Carbonsäuren wie Ameisensäure, Essigsäure, Propionsäure, Buttersäure, Isobuttersäure, Valeriansäure und Capronsäure.

Beispiele für geeignete Aldehyde **ALD2** der Formel (IV b) sind 2,2-Dimethyl-3-formyloxy-propanal, 3-Acetoxy-2,2-dimethylpropanal, 2,2-Dimethyl-3-propionoxypropanal, 3-Butyroxy-2,2-dimethyl-propanal, 2,2-Dimethyl-3-isobutyroxy-propanal, 2,2-Dimethyl-3-pentoyloxy-propanal, 2,2-Dimethyl-3-isopentoyloxypropanal und 2,2-Dimethyl-3-hexoyloxy-propanal.

Als Aldehyd **ALD** der Formel (IV) bevorzugt sind Aldehyde **ALD2** der Formel (IV b).

Besonders bevorzugt ist 3-Acetoxy-2,2-dimethylpropanal.

Die Aldehyde **ALD** der Formel (IV) stellen tertiäre aliphatische Aldehyde dar. Die Aldehyde **ALD,** insbesondere die Aldehyde **ALD2** der Formel (IV b), weisen einen deutlich geringeren Geruch auf als die im Stand der Technik üblicherweise zur Herstellung von Aldiminen eingesetzten Aldehyde, wie zum Beispiel Isobutyraldehyd oder Pivalaldehyd. Weiterhin lassen sich die Aldehyde **ALD,** insbesondere die Aldehyde **ALD2,** aus preiswerten Rohstoffen gut herstellen.

Für die Umsetzung zum Aldimin der Formel (I) geeignete Amine **B1** der Formel (III) enthalten eine oder mehrere aliphatische primäre Aminogruppen und mindestens eine Reaktivgruppe, welche einen aktiven Wasserstoff aufweist.

Als "aliphatische Aminogruppe" wird eine Aminogruppe bezeichnet, die an einen aliphatischen, cycloaliphatischen oder arylaliphatischen Rest gebunden ist. Sie unterscheidet sich damit von einer "aromatischen Aminogruppe", welche direkt an einen aromatischen oder heteroaromatischen Rest gebunden ist, wie beispielsweise in Anilin oder 2-Aminopyridin.

Als Amin **B1** der Formel (III) geeignet sind in einer Ausführungsform Verbindungen mit einer oder zwei primären aliphatischen und einer sekundären Aminogruppe, wie beispielsweise N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Amino-methyl-piperidin, 3-(4-Aminobutyl)-piperidin, N-(2-Aminoethyl)piperazin, Diethy-lentriamin (DETA), Bis-hexamethylentriamin (BHMT), 3-(2-Aminoethyl)amino-propylamin; Di- und Triamine aus der Cyanoethylierung oder Cyanobutylierung und anschliessender Hydrierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methyl-amino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecyl-amino-1-pentylamin, 3-Cyclohexylamino-1-pentylamin, Dipropylentriamin (DPTA), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen^{®} von Akzo Nobel erhältlich sind; die Produkte aus der Michael-artigen Addition von aliphatischen primären Di- oder Triaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1.

Als Amin **B1** der Formel (III) geeignet sind in einer weiteren Ausführungsform aliphatische Hydroxyamine, wie beispielsweise 2-Aminoethanol, 2-Methylaminoethanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykol-monoamin, α-(2-Hydroxymethylethyl)-ω-(2-aminomethylethoxy)-poly(oxy(methyl-1,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, beispielsweise 3-(2-Hydroxyethoxy)-propylamin, 3-(2-(2-Hydroxyethoxy)-ethoxy)-propylamin und 3-(6-Hydroxyhexyloxy)-propylamin.

Als Amin **B1** der Formel (III) geeignet sind in einer weiteren Ausführungsform aliphatische Mercaptoamine, wie beispielsweise 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dode-canthiol und Aminothiozucker wie 2-Amino-2-deoxy-6-thioglucose.

Als Amin **B1** der Formel (III) geeignet sind in einer weiteren Ausführungsform Amine, welche neben einer oder mehreren primären Aminogruppen mehrere weitere Reaktivgruppen enthaltend einen aktiven Wasserstoff aufweisen, beispielsweise die im Folgenden genannten Verbindungen:
- mehr als eine sekundäre Aminogruppe und eine oder mehrere primäre Aminogruppen tragende aliphatische Amine, wie N,N'-Bis-(3-amino-propyl)ethylendiamin, Triethylentetramin (TETA), Tetraethylenpentamin (TEPA), Pentaethylenhexamin und höhere Homologe linearer Polyethylenamine, N,N'-Bis-(3-aminopropyl)-ethylendiamin, Polyamine aus der mehrfachen Cyanoethylierung oder Cyanobutylierung und anschliessender Hydrierung von primären Di- und Polyaminen mit mehreren primären Aminogruppen, wie N,N'-Bis-(3-aminopropyl)-ethylendiamin, N,N'-Bis-(3-amino-propyl)-1,4-diaminobutan, N,N'-Bis-(3-aminopropyl)-2-methyl-1,5-pentandiamin, N,N'-Bis-(3-amino-1-ethylpropyl)-2-methyl-1,5-pentandiamin, Polyvinyl-amine, sowie Polyethylenimine unterschiedlichen Polymerisationsgrades (Molmassen-Bereich 500 bis 1'000'000 g/mol), wie sie beispielsweise unter dem Handelsnamen Lupasol^{®} von BASF in reiner Form oder als wässrige Lösungen erhältlich sind, wobei diese Polyethylenimine neben primären und sekundären auch tertiäre Aminogruppen enthalten.
- mehr als eine Hydroxylgruppe und eine oder mehrere primäre Aminogruppen tragende Hydroxyamine, insbesondere Derivate von polyalkoxylierten drei- oder höherwertigen Alkoholen oder von polyalkoxylierten Polyaminen, sowie Aminozucker, beispielsweise Glucosamin oder Galactosamin;
- mindestens eine Hydroxyl- und mindestens eine sekundäre Aminogruppe tragende Hydroxypolyamine aus der Cyanoethylierung oder Cyanobutylierung und anschliessender Hydrierung von Hydroxyaminen wie N-Hydroxyethyl-1,2-ethandiamin, N-Hydroxypropyl-1,2-ethandiamin, N-Hydroxyethyl-1,3-propandiamin, N3-Hydroxyethyl-1,3-pentandiamin.

Als Amin **B1** insbesondere geeignet sind Amine, welche abgesehen von den primären Aminogruppen nur einen aktiven Wasserstoff aufweisen, also Amine **B1**, bei welchen der Index y in Formel (III) für 1 steht.

Als Amin **B1** bevorzugt sind N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin; Produkte aus der Michael-artigen Additionsreaktion von aliphatischen primären Diaminen mit Malein- und Fumarsäurediestern, Acryl- und Methacrylsäureestern, Acryl- und Methacrylsäureamiden, bevorzugt mit Maleinsäurediestern, insbesondere Maleinsäuredimethyl-, -diethyl-, -dipropyl- und -dibutylester, und mit Acrylsäureestern, insbesondere Acrylsäuremethylester, umgesetzt im Molverhältnis 1:1; sowie aliphatische Hydroxy- oder Mercaptoamine, in denen die primäre Aminogruppe von der Hydroxyl- oder Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Amino-methyl-3,5,5-trimethyl-cyclohexanol, 2-(2-Aminoethoxy)-ethanol, Triethylen-glykol-monoamin und höhere Oligo- und Polymere davon, 3-(2-Hydroxy-ethoxy)-propylamin, 3-(2-(2-Hydroxyethoxy)-ethoxy)-propylamin und 3-(6-Hydroxyhexyloxy)-propylamin.

Als Amin **B1** besonders bevorzugt sind Amine, welche ausgewählt sind aus der Gruppe bestehend aus N-Methyl-1,2-ethandiamin, N-Ethyl-1,2-ethan-diamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT, Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin.

Die Umsetzung zwischen einem Aldehyd ALD und einem Amin **B1** führt dabei zu Hydroxyaldiminen, wenn als Amin **B1** ein Hydroxyamin eingesetzt wird; zu Mercaptoaldiminen, wenn als Amin **B1** ein Mercaptoamin eingesetzt wird; oder zu Aminoaldiminen, wenn als Amin **B1** ein zwei- oder mehrwertiges Amin, welches neben einer oder mehreren primären Aminogruppen eine oder mehrere sekundäre Aminogruppen trägt, eingesetzt wird.

In einer Ausführungsform des Aldimins der Formel (I) steht X für N-R⁶. Derartige Aldimine der Formel (I) lassen sich gegebenenfalls auf einem leicht anderen Syntheseweg als dem bisher beschriebenen herstellen. Dieser Syntheseweg besteht darin, dass ein Aldehyd **ALD** der Formel (IV) mit einem zweiwertigen aliphatischen primären Amin **B2** der Formel H₂N-A^{a}-NH₂ - wobei A^{a} für einen zweiwertigen, gegebenenfalls Heteroatome aufweisenden, Kohlenwasserstoffrest mit 2 bis 20 C-Atomen steht - in einem ersten Schritt zu einem Zwischenprodukt umgesetzt wird, welches neben einer Aldiminogruppe noch eine primäre Aminogruppe enthält. Dieses Zwischenprodukt wird anschliessend in einem zweiten Schritt zu einem Aldimin der Formel (I) umgesetzt, indem die primäre Aminogruppe einfach alkyliert wird. Für die Alkylierung werden insbesondere Verbindungen mit nur einer aktivierten Doppelbindung eingesetzt, welche mit primären Aminen Michael-artige Additionsreaktionen eingehen können; solche Verbindungen werden im Folgenden als "Michael-Akzeptor" bezeichnet.

Die Umsetzung eines Aldehyds **ALD** mit einem Amin **B2** zum eine primäre Aminogruppe aufweisenden Zwischenprodukt erfolgt in einer Kondensationsreaktion unter Abspaltung von Wasser, wie sie weiter oben für die Umsetzung mindestens eines Aldehyds **ALD** mit mindestens einem Amin **B1** der Formel (III) beschrieben wird. Die Stöchiometrie zwischen dem Aldehyd **ALD** und dem Amin **B2** wird dabei so gewählt, dass 1 mol Aldehyd **ALD** für 1 mol Amin **B2,** welches zwei primäre Aminogruppen enthält, eingesetzt wird. Es wird ein lösemittelfreies Herstellverfahren bevorzugt, wobei das bei der Kondensation gebildete Wasser mittels Anlegen von Vakuum aus der Reaktionsmischung entfernt wird.

Die Umsetzung des eine primäre Aminogruppe aufweisenden Zwischenprodukts mit dem Michael-Akzeptor erfolgt beispielsweise dadurch, dass das Zwischenprodukt mit einer stöchiometrischen oder leicht überstöchiometrischen Menge des Michael-Akzeptors gemischt wird und die Mischung bei Temperaturen von 20 bis 110 °C bis zum vollständigen Umsatz des Zwischenprodukts zum Aldimin der Formel (I) erwärmt wird. Die Umsetzung erfolgt bevorzugt ohne die Verwendung von Lösemitteln.

Beispiele für geeignete Amine **B2** sind aliphatische Diamine wie Ethylendiamin, 1,2- und 1,3-Propandiamin, 2-Methyl-1,2-propandiamin, 2,2-Dimethyl-1,3-propandiamin, 1,3- und 1,4-Butandiamin, 1,3- und 1,5-Pentandiamin, 2-Butyl-2-ethyl-1,5-pentandiamin, 1,6-Hexamethylendiamin (HMDA), 2,2,4- und 2,4,4-Trimethylhexamethylendiamin und Mischungen davon (TMD), 1,7-Heptandiamin, 1,8-Octandiamin, 2,4-Dimethyl-1,8-octandiamin, 4-Amino-methyl-1,8-octandiamin, 1,9-Nonandiamin, 2-Methyl-1,9-nonandiamin, 5-Methyl-1,9-nonandiamin, 1,10-Decandiamin, Isodecandiamin, 1,11-Undecandiamin, 1,12-Dodecandiamin, Methyl-bis-(3-aminopropyl)amin, 1,5-Diamino-2-methylpentan (MPMD), 1,3-Diaminopentan (DAMP), 2,5-Dimethyl-1,6-hexamethylendiamin; cycloaliphatische Diamine wie 1,2-, 1,3- und 1,4-Diamino-cyclohexan, Bis-(4-aminocyclohexyl)-methan (H₁₂MDA), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 1-Cyclohexyl-amino-3-aminopropan, 2,5(2,6)-Bis-(aminomethyl)-bicyclo[2.2.1]heptan (NBDA, hergestellt von Mitsui Chemicals), 3(4),8(9)-Bis-(aminomethyl)-tricyclo-[5.2.1.0^{2,6}]decan, 1,4-Diamino-2,2,6-trimethylcyclohexan (TMCDA), 3,9-Bis-(3-aminopropyl)-2,4,8,10-tetraoxaspiro[5.5]undecan; arylaliphatische Diamine wie 1,3-Xylylendiamin (MXDA), 1,4-Xylylendiamin (PXDA); Ethergruppen-haltige aliphatische Diamine wie Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin und höhere Oligomere dieser Diamine, sowie Polyoxyalkylen-Diamine. Letztere stellen typischerweise Produkte aus der Aminierung von Polyoxyalkylen-Diolen dar und sind beispielsweise erhältlich unter dem Namen Jeffamine^{®} (von Huntsman Chemicals), unter dem Namen Polyetheramin (von BASF) oder unter dem Namen PC Amine^{®} (von Nitroil). Insbesondere geeignete Polyoxyalkylen-Diamine sind Jeffamine^{®} D-230, Jeffamine^{®} D-400, Jeffamine^{®} XTJ-511, Jeffamine^{®} XTJ-568 und Jeffamine^{®} XTJ-569; Polyetheramin D 230 und Polyetheramin D 400; PC Amine^{®} DA 250 und PC Amine^{®} DA 400.

Als Amin B2 bevorzugt sind solche Diamine, in denen die primären Aminogruppen durch eine Kette von mindestens 5 Atomen, oder durch einen Ring, getrennt sind, insbesondere 1,5-Diamino-2-methylpentan, 1,6-Hexa-methylendiamin, 2,2,4- und 2,4,4-Trimethylhexamethylendiamin und Mischungen davon, 1,10-Decandiamin, 1,12-Dodecandiamin, 1,3- und 1,4-Diamino-cyclohexan, Bis-(4-aminocyclohexyl)-methan, Bis-(4-amino-3-methylcyclohexyl)-methan, 1-Amino-3-aminomethyl-3,5,5-trimethylcyclohexan, 1,3- und 1,4-Bis-(aminomethyl)cyclohexan, 2,5(2,6)-Bis-(aminomethyl)-bicyclo[2.2.1]-heptan, 3(4),8(9)-Bis-(aminomethyl)-tricyclo[5.2.1.0^{2,6}]decan, 1,4-Diamino-2,2,6-trimethylcyclohexan (TMCDA), 3,9-Bis-(3-aminopropyl)-2,4,8,10-tetraoxaspiro[5.5]undecan, 1,3- und 1,4-Xylylendiamin, sowie die genannten Ethergruppen-haltigen aliphatischen Diamine und Polyoxyalkylen-Diamine.

Beispiele für geeignete Michael-Akzeptoren sind Malein- oder Fumarsäurediester wie Dimethylmaleinat, Diethylmaleinat, Dibutylmaleinat, Diethylfumarat; Citraconsäurediester wie Dimethylcitraconat; Acryl- oder Methacrylsäureester wie Methyl(meth)acrylat, Ethyl(meth)acrylat, Butyl(meth)acrylat, Lauryl(meth)acrylat, Stearyl(meth)acrylat, Tetrahydrofuryl(meth)acrylat, Isobornyl(meth)acrylat; Itaconsäurediester wie Dimethylitaconat; Zimtsäureester wie Methylcinnamat; Vinylphosphonsäurediester wie Vinylphosphonsäuredimethylester; Vinylsulfonsäureester, insbesondere Vinylsulfonsäurearylester; Vinylsulfone; Vinylnitrile wie Acrylnitril, 2-Pentennitril oder Fumaronitril; 1-Nitro-ethylene wie β-Nitrostyrol, und Knoevenagel-Kondensationsprodukte, wie beispielsweise solche aus Malonsäurediestern und Aldehyden wie Formaldehyd, Acetaldehyd oder Benzaldehyd. Bevorzugt sind Maleinsäurediester, Acrylsäureester, Phosphonsäurediester und Vinylnitrile.

Die Aldimine der Formel (I) können gegebenenfalls im Gleichgewicht stehen mit cyclischen Formen, wie sie in Formel (V) für den Fall y = 1 beispielhaft gezeigt sind. Diese cyclischen Formen sind im Fall von Aminoaldiminen cyclische Aminale, beispielsweise Imidazolidine oder Tetrahydropyrimidine; im Fall von Hydroxyaldiminen cyclische Aminoacetale, beispielsweise Oxazolidine oder Tetrahydrooxazine; im Fall von Mercaptoaldiminen cyclische Thioaminale, beispielsweise Thiazolidine oder Tetrahydrothiazine.

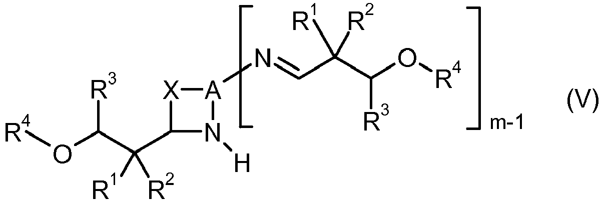

In der Formel (V) haben R¹, R², R³, R⁴, A, X und m die gleiche Bedeutung wie für Formel (I) beschrieben.

Überraschenderweise neigen die meisten Aldimine der Formel (I) nicht zur Cyclisierung. Insbesondere für Aminoaldimine kann mittels IR- und NMRspektroskopischen Methoden gezeigt werden, dass diese Verbindungen überwiegend in der offenkettigen, also der Aldimin-Form, vorliegen, während die cyclische, also die Aminal-Form, nicht oder nur in Spuren vorkommt. Dies steht im Gegensatz zum Verhalten der Aminoaldimine nach dem Stand der Technik, wie sie beispielsweise in US 6,136,942 beschrieben werden: jene liegen nämlich hauptsächlich in der Cycloaminal-Form vor. Auch Hydroxyl- und Mercaptoaldimine, in denen die primäre Aminogruppe von der Hydroxylbeziehungsweise der Mercaptogruppe durch eine Kette von mindestens 5 Atomen, oder durch einen Ring, getrennt sind, zeigen kaum Cyclisierung. Die weitgehende Abwesenheit cyclischer Strukturen in den Aldiminen der Formel (I) ist, insbesondere im Hinblick auf deren Verwendung in Isocyanat-haltigen Zusammensetzungen, als Vorteil zu werten, da die Aldimine dadurch weitgehend frei sind von den in Aminalen, Oxazolidinen und Thioaminalen vorkommenden basischen Stickstoffatomen, welche die Lagerstabilität der Isocyanat-haltigen Zusammensetzung herabsetzen können.

Die Aldimine der Formel (I) sind unter geeigneten Bedingungen, insbesondere unter Ausschluss von Feuchtigkeit, lagerstabil. Bei Zutritt von Feuchtigkeit können ihre Aldiminogruppen über Zwischenstufen formal zu Aminogruppen hydrolysieren, wobei der entsprechende, zur Herstellung des Aldimins der Formel (I) verwendete, Aldehyd ALD freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen hydrolysieren.

Die Aldimine der Formel (I) weisen bezüglich ihrer Aldiminogruppen eine tertiäre Struktur auf. Sie weisen also am C-Atom, welches in alphaStellung zur Aldiminogruppe steht, keine Wasserstoffatome auf. Die Aldiminogruppen können deshalb nicht zu Enaminogruppen tautomerisieren und sind sterisch gehindert. Aufgrund dieser Eigenschaften verhalten sie sich in unhydrolysierter Form inert gegenüber den meisten Additionsreaktionen eingehenden Verbindungen, beispielsweise Epoxiden und Isocyanaten.

Die Aldimine der Formel (I) weisen gegenüber bekannten Aldiminen mit tertiärer Struktur, wie beispielsweise Aldiminen abgeleitet von Pivalaldehyd, einen geringeren Geruch auf, was für viele Anwendungen, insbesondere Anwendungen in geschlossenen Räumen oder Anwendungen, bei welchen die Zusammensetzungen gespritzt oder bei erhöhter Temperatur appliziert werden, ein grosser Vorteil ist. Sie sind gut verträglich mit Polymersystemen, insbesondere Polyurethanen, sowie aus preiswerten Rohstoffen in einem einfachen Verfahren gut herstellbar.

Die Aldimine der Formel (I) lassen sich sehr breit verwenden. Grundsätzlich lassen sie sich überall dort einsetzen, wo sie als Quelle von Aldehyden **ALD** der Formel (IV) oder von Aminen **B1** der Formel (III) dienen können. Insbesondere können sie in der Funktion von geschützten Aminen, beziehungsweise geschützten Aldehyden, in Aldehyd- und/oder oder Amin-reaktiven Systemen eingesetzt werden und dort bei Bedarf gezielt entschützt werden. Insbesondere finden sie in Systemen Anwendungen, in welchen Verbindungen vorhanden sind, welche mit primären Aminen reagieren. Die Entschützung erfolgt hydrolytisch, beispielsweise durch Kontakt mit Wasser oder Feuchtigkeit, insbesondere Luftfeuchtigkeit.

Andererseits finden die Aldimine der Formel (I) Verwendung beim Aufbau von weiter funktionalisierten Reaktionsprodukten der Aldimine der Formel (I). So können Aldimine der Formel (I) mit Verbindungen, welche mit der aktiven Wasserstoff tragenden Gruppe HX reagieren können, umgesetzt werden, insbesondere in Additionsreaktionen. Geeignete solche Additionsreaktionen eingehende Verbindungen tragen Reaktivgruppen wie zum Beispiel Isocyanatgruppen, Epoxidgruppen, Silangruppen, Anhydridgruppen oder mehr oder weniger stark aktivierte Doppel- oder Dreifachbindungen wie (Meth)acrylatgruppen, Acrylamidgruppen, 1-Ethinylcarbonyl- oder 1-Propinylcarbonylgruppen, Maleimid- oder Citraconimidgruppen, Vinyl- oder Isopropenylgruppen oder Allylgruppen. Die Aldiminogruppen tragenden Umsetzungsprodukte aus solchen Additionsreaktionen lassen sich bei Bedarf zu Aldehyden **ALD** der Formel (IV) und Verbindungen mit primären Aminogruppen hydrolysieren und darauf für weitere Reaktionen nutzen, beispielsweise zu Vernetzungsreaktionen.

Durch Reduktion der Aldiminogruppen zu sekundären Aminogruppen können die Aldimine der Formel (I), oder die erwähnten Umsetzungsprodukte aus Additionsreaktionen mit Aldiminen der Formel (I), auch zum Aufbau von Verbindungen enthaltend spezielle sekundäre Aminogruppen dienen, welche ihrerseits beispielsweise als Härter für Isocyanat- und/oder Epoxid-haltige Zusammensetzungen eingesetzt werden können.

Weiterhin können die Aldimine der Formel (I) zur Herstellung von Aldiminogruppen-haltigen Verbindungen eingesetzt werden, welche beispielsweise als latente Härter oder als Co-Monomere für reaktive Polymerzusammensetzungen, insbesondere für Isocyanatgruppen aufweisende Polyurethanzusammensetzungen, geeignet sind.

Besonders geeignet sind die Aldimine der Formel (I) als Bestandteil von Zusammensetzungen basierend auf Isocyanaten oder Epoxidharzen, insbesondere für Anwendungen wie Klebstoffe, Dichtstoffe, Vergussmassen, Beschichtungen, Bodenbeläge, Anstriche, Lacke, Primer oder Schäume. Bevorzugt enthalten solche Zusammensetzungen mindestens eine Säure, insbesondere eine organische Carbon- oder Sulfonsäure, oder eine zu diesen Säuren hydrolysierbare Verbindung, wobei die Säure die Hydrolyse der Aldiminogruppen katalysiert.

Insbesondere eignen sich die Aldimine der Formel (I) als Härter oder als Vorläufer für Härter für ein- und zweikomponentige feuchtigkeitshärtende Polyurethanzusammensetzungen wie Klebstoffe, Dichtstoffe, Vergussmassen, Beschichtungen, Bodenbeläge, Anstriche, Lacke, Primer oder Schäume, sowie zur Herstellung von rasch aushärtenden reaktiven Polyurethan-Heissschmelzklebstoffen, welche einen niedrigen Gehalt an Isocyanat-Monomeren aufweisen.

Die in einer solchen Zusammensetzung vorhandenen Isocyanatgruppen reagieren bei Zutritt von Feuchtigkeit mit den durch Hydrolyse der Aldiminogruppen der Aldimine der Formel (I) formal frei werdenden primären Aminogruppen, wobei ein Aldehyd **ALD** freigesetzt wird. Im Verhältnis zu den Aldiminogruppen überschüssige Isocyanatgruppen reagieren direkt mit Feuchtigkeit. Als Ergebnis dieser Reaktionen härtet die Zusammensetzung schliesslich aus; dieser Prozess wird auch als Vernetzung bezeichnet. Die Reaktion der Isocyanatgruppen mit den hydrolysierenden Aldiminogruppen muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert.

Ein weiterer Gegenstand der vorliegenden Erfindung sind Aldiminogruppen-haltige Verbindungen **AV,** welche Additionsprodukte darstellen aus der Umsetzung von mindestens einem Aldimin der Formel (I) mit mindestens einer Verbindung **D,** die mehr als eine Reaktivgruppe trägt, welche mit einer Gruppe HX Additionsreaktionen eingehen kann. Die Gruppen HX des Aldimins der Formel (I) reagieren dabei in einer Additionsreaktion mit mindestens einer Reaktivgruppe der Verbindung D zu einer Aldiminogruppen-haltigen Verbindung **AV.**

Für diese Umsetzung wird bevorzugt ein Aldimin der Formel (I) eingesetzt, in dem der Index y für den Wert 1 steht.

Wird diese Umsetzung stöchiometrisch geführt, das heisst mit einem Molequivalent aktivem Wasserstoff des Aldimins der Formel (I) auf ein Molequivalent Reaktivgruppen der Verbindung **D -** wodurch deren Reaktivgruppen vollständig umgesetzt werden -, wird als Aldiminogruppen-haltige Verbindung **AV** ein Polyaldimin erhalten. Auf einfache Weise sind so Polyaldimine erhältlich, ohne dass zu ihrer Herstellung auf die entsprechenden primären Polyamine, welche technisch und kommerziell nur beschränkt zur Verfügung stehen, zurückgegriffen werden müsste. In Abhängigkeit von Struktur, Funktionalität und Molekulargewicht der Verbindungen **D** und der Aldimine der Formel (I) können diese Polyaldimine höchst unterschiedliche Eigenschaften aufweisen; sie lassen sich also auf die Bedürfnisse einer bestimmten Anwendung massschneidern. Diese Polyaldimine eignen sich insbesondere als latente Härter für Isocyanatgruppen aufweisende Zusammensetzungen.

Durch eine unterstöchiometrische Umsetzung der Aldimine der Formel (I) mit Verbindungen **D** können auch Aldiminogruppen-haltige Verbindungen **AV** hergestellt werden, welche neben einer oder mehreren Aldiminogruppen noch eine oder mehrere andere, für Polyreaktionen zugängliche Reaktivgruppen aufweisen, sogenannte heterofunktionelle Verbindungen **AV.** Dabei wird weniger als ein Molequivalent aktiver Wasserstoff des Aldimins der Formel (I) auf ein Molequivalent Reaktivgruppen der Verbindung **D** eingesetzt. Die Verbindung **D** selbst kann dabei homo- oder heterofunktionell sein. Solche heterofunktionellen Verbindungen **AV** sind beispielsweise verwendbar als Co-Monomere oder als latente Härter für reaktive Polymerzusammensetzungen; oder, für den Fall, dass eine heterofunktionelle Verbindung **AV** neben mindestens einer Aldiminogruppe mindestens eine Reaktivgruppe aufweist, welche mit hydrolysierenden Aldiminogruppen unter Verknüpfung der Moleküle reagieren kann, auch als reaktive Polymerzusammensetzung selbst. Dies trifft insbesondere für den Fall von Aldiminogruppen-haltigen Verbindungen **AV** zu, die zusätzlich Isocyanatgruppen aufweisen.

Als Verbindungen **D** geeignet sind Substanzen, bei welchen die Reaktivgruppen der Verbindung **D** ausgewählt sind aus der Gruppe bestehend aus Isocyanat-, Isothiocyanat-, Cyclocarbonat-, Epoxid-, Episulfid-, Aziridin-, Acryl-, Methacryl-, 1-Ethinylcarbonyl-, 1-Propinylcarbonyl-, Maleimid-, Citraconimid-, Vinyl-, Isopropenyl- und Allyl-Gruppen, wobei die einzelnen Reaktivgruppen gleich oder verschieden voneinander sein können. Bevorzugt sind Isocyanat-, Epoxid-, Acryl-, Maleimid-, Vinyl-, Isopropenyl- und Allylgruppen.

Beispiele für geeignete Verbindungen **D** sind
- zwei- oder mehrwertige, monomere und/oder oligomere aliphatische, cycloaliphatische, arylaliphatische und aromatische Polyisocyanate wie 1,6-Hexamethylendiisocyanat (HDI), 2-Methylpentamethylen-1,5-diisocyanat, 2,2,4-und 2,4,4-Trimethyl-1,6-hexamethylendiisocyanat (TMDI), 1,12-Dodecamethylendiisocyanat, Lysin- und Lysinesterdiisocyanat, Cyclohexan-1,3- und-1,4-diisocyanat und beliebige Gemische dieser Isomeren, 1-Isocyanato-3,3,5trimethyl-5-isocyanatomethyl-cyclohexan (=Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3- und 1,4-Bis-(isocyanatomethyl)-cyclo-hexan, m- und p-Xylylendiisocyanat (m- und p-XDI), 1,3,5-Tris-(isocyanato-methyl)-benzol, m- und p-Tetramethyl-1,3- und -1,4-xylylendiisocyanat (m- und p-TMXDI), Bis-(1-Isocyanato-1-methylethyl)-naphthalin, α,α,α',α',α",α"-Hexa-methyl-1,3,5-mesitylentriisocyanat, Dimer- und Trimerfettsäureisocyanate wie 3,6-Bis-(9-isocyanatononyl)-4,5-di-(1-heptenyl)-cyclohexen (Dimeryldiisocyanat), 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diiso-cyanatodiphenyl (TODI), Tris-(4-isocyanatophenyl)-methan, Tris-(4-isocyanatophenyl)-thiophosphat; Oligomere dieser Isocyanate enthaltend Uretdion-, Isocyanurat- oder Iminooxadiazindiongruppen; modifizierte zwei- und mehrwertige Isocyanate enthaltend Ester-, Harnstoff-, Urethan-, Biuret-, Allophanat-, Carbodiimid-, Uretonimin- oder Oxadiazintriongruppen; sowie Isocyanat-haltige Polyurethanpolymere, das heisst mehr als eine Isocyanatgruppe aufweisende Umsetzungsprodukte von Polyisocyanaten mit zwei- oder mehr Hydroxylgruppen aufweisenden Substanzen (so genannten "Polyolen"), wie beispielsweise zwei- oder mehrwertigen Alkoholen, Glykolen oder Aminoalkoholen, polyhydroxyfunktionellen Polyethern, Polyestern, Polyacrylaten, Polycarbonaten oder Polykohlenwasserstoffen, insbesondere Polyethern;
- zwei- oder mehrwertige Epoxide (Polyepoxide) wie Bis-(2,3-epoxycyclopentyl)ether, Polyglycidylether von mehrwertigen aliphatischen und cycloaliphatischen Alkoholen wie 1,4-Butandiol, Polypropylenglykolen und 2,2-Bis-(4-hydroxycyclohexyl)-propan; Polyglycidylether von mehrwertigen Phenolen wie Resorcinol, Bis-(4-hydroxyphenyl)-methan (Bisphenol-F), 2,2-Bis-(4-hydroxyphenyl)-propan (Bisphenol-A), 2,2-Bis-(4-hydroxy-3,5-dibromophenyl)-propan, 1,1,2,2-Tetrakis-(4-hydroxyphenyl)-ethan, Kondensationsprodukten von Phenolen mit Formaldehyd, die unter sauren Bedingungen erhalten werden, wie Phenolnovolaken und Kresolnovolaken, sowie mit diesen Alkoholen und Phenolen, oder mit Polycarbonsäuren wie beispielsweise dimeren Fettsäuren, oder einem Gemisch davon, vorverlängerte Polyglycidylether; Polyglycidylester von mehrwertigen Carbonsäuren wie Phthalsäure, Terephthalsäure, Tetrahydrophthalsäure und Hexahydrophthalsäure; N-Glycidyl-Derivate von Aminen, Amiden und heterocyclischen Stickstoffbasen wie N,N-Diglycidylanilin, N,N-Diglycidyltoluidin, N,N,O-Triglycidyl-4-aminophenol, N,N,N',N'-Tetraglycidyl-bis-(4-aminophenyl)-methan, Triglycidylcyanurat und Triglycidylisocyanurat;
- zwei- oder mehrwertige, Acryl-, Methacryl- oder Acrylamidgruppen tragende Verbindungen wie Tris-(2-hydroxyethyl)-isocyanurat-tri(meth)acrylat, Tris-(2-hydroxyethyl)-cyanurat-tri(meth)acrylat, N,N',N"-Tris-(meth)acryloyl-per-hydrotriazin; zwei- oder mehrwertige Acrylate und Methacrylate von aliphatischen Polyethern, Polyestern, Novolaken, Phenolen, aliphatischen oder cycloaliphatischen Alkoholen, Glykolen und Polyesterglykolen sowie mono- und polyalkoxylierten Derivaten der vorgenannten Verbindungen, beispielsweise Ethylenglykol-di(meth)acrylat, Tetraethylenglykol-di(meth)acrylat, Tripropylenglykol-di(meth)acrylat, Polyethylenglykol-di(meth)acrylat, Polypropylenglykol-di(meth)acrylat, 1,4-Butandiol-di(meth)acrylat, 1,6-Hexandiol-di(meth)acrylat, Neopentylglykol-di(meth)acrylat, Trimethylolpropan-tri(meth)acrylat, Pentaerithritol-tetra(meth)acrylat, Dipentaerithritol-tetra(meth)acrylat, Dipentaerithritolpenta(meth)acrylat, Dipentaerithritol-hexa(meth)acrylat; zwei- oder mehrwertige Acryl- oder Methacryl-funktionelle Polybutadiene, Polyisoprene oder Block-copolymere davon; Addukte aus zwei- oder mehrwertigen Epoxiden wie die oben genannten Epoxide mit Acryl- und Methacrylsäure; zwei- oder mehrwertige Polyurethan(meth)acrylate; zwei- oder mehrwertige Acrylamide wie N,N'-Methylen-bis-acrylamid;

- zwei- oder mehrwertige 1-Ethinylcarbonyl- oder 1-Propinylcarbonylgruppen tragende Verbindungen;
- zwei- oder mehrwertige Maleimid- oder Citraconimidgruppen tragende Verbindungen wie die Bis- und Polykismaleimide aus aliphatischen, cyclo-aliphatischen oder aromatischen Di- und Polyaminen und Malein- oder Citraconsäureanhydrid, beispielsweise α,ω-Dimerfettsäure-bis(maleimid), 4,4'-Di-phenylmethan-bis(maleimid), 1,3-Xylylen-bis(citraconimid); Bis- und Polykismaleimide aus aminoterminierten Butadien/Acrylonitril-Copolymeren (beispielsweise erhältlich unter dem Namen Hycar^{®} ATBN von Noveon) und Malein- oder Citraconsäureanhydrid; zwei- oder mehrwertige Addukte aus Di- und Polyisocyanaten mit N-Hydroxyethylmaleimid; Ester aus zwei- oder mehrwertigen Alkoholen und 6-Maleimidohexansäure;
- zwei- oder mehrwertige Vinyl- und/oder Isopropenylgruppen tragende Verbindungen wie 1,3- und 1,4-Divinylbenzol, Divinylsulfon, Vinylcrotonat, Diallylidenpentaerythritol-acetal, 1,3-Diisopropenylbenzol und 1,3,5-Triisopropenyl-benzol, 3-(2-Vinyloxyethoxy)-styrol, Divinyldimethylsilan, Trivinylmethylsilan, Trivinylmethoxysilan, Divinyltetramethyldisiloxan, 1,3-Divinyl-1,3-diphenyl-1,3-dimethyldisiloxan, 1,3-Divinyl-tetraethoxydisiloxan, Trivinyl-pentamethyltrisiloxan, 4-Vinyloxybutoxy-trivinylsilan, Tris-(4-vinyloxybutoxy)-vinylsilan; zwei- oder mehrwertige Vinyl- und Isopropenylether wie Divinylether, Isopropenylvinylether, Triethylenglykol-divinylether, Butandiol-divinylether, Hexandiol-divinylether, Octadecandiol-divinylether, Dimerfettsäurediol-divinylether und Divinylbutyral; Divinylester von Dicarbonsäuren, beispielsweise Adipinsäuredivinylester;

- zwei- oder mehrwertige Allylgruppen tragende Verbindungen wie Triallylcyanurat, Triallylisocyanurat, Triallylphosphat; zwei- oder mehrwertige Allylether von Alkoholen und Glykolen sowie mono- und polyalkoxylierten Derivaten davon, beispielsweise 1,4-Bis-(allyloxy)-butan, 1,6-Bis-(allyloxy)-hexan, Triethylenglykol-diallylether, Bisphenol-A-diallylether, 3,3'-Diallyl-bisphenol-A-diallylether, 3,3'-Diallyl-bisphenol-A, Trimethylolpropan-diallylether, Glycerin-triallylether, Trimethylolpropan-triallylether, Pentaerithritol-tetraallylether; zwei- oder mehrwertige Allylester und -amide von Carbonsäuren, beispielsweise Diallylphthalat, Diallyliso- und -terephthalat, Diallyloxalat, Diallylsebacat, Diallylmaleinat, Diallylfumarat, Diallylitaconat; zweiwertige Allylcarbonate wie Diallylcarbonat, Di- und Triethylenglykol-bis-allylcarbonat; zwei- oder mehrwertige Addukte aus Di- und Polyisocyanaten mit Glycidol, Allylalkohol oder Allylglykolen, beispielsweise 1,6-Hexamethylen-bis-allylcarbamat;
- sowie zwei- oder mehrwertige heterofunktionelle, das heisst mindestens zwei verschiedene der vorgenannten Reaktivgruppen tragende, Verbindungen wie 4-Allyloxy-phenylisocyanat, 1-Alkenylisocyanate wie Vinylisocyanat, Propenylisocyanat und Isopropenylisocyanat, 2-Isocyanatoethyl-methacrylat, 1,2-Dimethyl-3-isocyanatopropyl-acrylat, p-Isocyanatostyrol, m- und p-Iso-propenyl-α,α-dimethylbenzylisocyanat (m- und p-TMI), m- und p-Ethenyl-α,α-dimethylbenzylisocyanat, Isopropenyl-α,α,α',α'-tetramethyl-xylylendiisocyanat, Glycidylallylether, Glycidoxy-trivinylsilan, Triglycidoxy-vinylsilan, N-(Trivinylsilyl-oxymethyl)-maleimid; heterofunktionelle Addukte aus Di- und Polyisocyanaten mit Glycidol, Allylalkohol, Allylglykolen, N-Hydroxyethylmaleimid, hydroxyfunktionellen Acrylat- und Methacrylaten wie 2-Hydroxyethyl-acrylat und - methacrylat; heterofunktionelle Addukte aus Mono- und Polycarbodiimiden von Di- und Polyisocyanaten mit Acryl- oder Methacrylsäure; heterofunktionelle Addukte aus zwei- oder mehrwertigen Epoxiden mit Acryl- oder Methacrylsäure, Vinylallylether, Ethylenglykol-vinylallylether, Vinylallylphthalat, Ethylenglykol-(2-allylphenyl)-vinyl-ether, Allyl(meth)acrylat, Vinylacrylat, 2-Vinyloxy-ethyl-(meth)acrylat.

Als Verbindungen D insbesondere geeignet sind zwei- oder mehrwertige aliphatische, cycloaliphatische, arylaliphatische und aromatische Isocyanate wie die erwähnten monomeren und oligomeren Polyisocyanate sowie die mehr als eine Isocyanatgruppe aufweisenden Umsetzungsprodukte von Polyisocyanaten mit Polyolen, insbesondere Polyetherpolyolen, Polyesterpolyolen, Polyacrylatpolyolen, Polycarbonatpolyolen, Polykohlenwasserstoffpolyolen und Mischungen dieser Polyole.

Je nach den Reaktivgruppen der Verbindung D und den aktiven Wasserstoff tragenden Gruppen des Aldimins der Formel (I) kann die zur Aldiminogruppen-haltige Verbindung AV führende Additionsreaktion nucleophil oder radikalisch erfolgen. Aus Gründen der Einfachheit soll der Begriff "Additionsreaktion" im vorliegenden Dokument auch ringöffnende Substitutionsreaktionen, wie sie beispielsweise Epoxide mit Nucleophilen eingehen, umfassen, weil das Ergebnis einer solchen, das Nucleofug nicht als separates Molekül freisetzenden, Substitutionsreaktion dem einer Additionsreaktion gleichkommt. Die Additionsreaktion verläuft nucleophil, wenn die den aktiven Wasserstoff tragende Reaktivgruppe des Aldimins als Nucleophil an eine elektrophile Reaktivgruppe der Verbindung **D** angreift, beispielsweise im Fall des Angriffs einer Amino- oder Hydroxylgruppe an eine Isocyanatgruppe. Als Beispiel für eine radikalische Additionsreaktion kann die Reaktion einer Mercaptogruppe an eine Acrylgruppe genannt werden, wobei für diese Art der Additionsreaktion im Allgemeinen ein radikalbildender Initiator benötigt wird.

Die Umsetzung zwischen dem Aldimin der Formel (I) und der Verbindung **D** zur Aldiminogruppen-haltige Verbindung **AV** erfolgt unter bekannten Bedingungen, wie sie für Reaktionen zwischen den an der jeweiligen Umsetzung beteiligten Reaktivgruppen typischerweise verwendet werden, beispielsweise bei 20 bis 100 °C. Die Umsetzung erfolgt unter Verwendung eines Lösemittels oder bevorzugt lösemittelfrei. Gegebenenfalls können Hilfsstoffe wie beispielsweise Katalysatoren, Initiatoren oder Stabilisatoren mitverwendet werden. Die Umsetzung mit Isocyanaten wird für Aminoaldimine bevorzugt bei Raumtemperatur und ohne Katalysator, für Hydroxy- und Mercaptoaldimine bei 40 bis 100 °C und unter Verwendung eines Katalysators, wie er für die Urethanisierungsreaktion zwischen Isocyanaten und Alkoholen verwendet wird, beispielsweise einer Organozinn-Verbindung, eines Bismutkomplexes, einer tertiären Aminverbindung oder einer Kombination solcher Katalysatoren, durchgeführt.

Als "Raumtemperatur" wird im vorliegenden Dokument eine Temperatur von 25 °C bezeichnet.

Die auf die beschriebene Weise erhaltenen Aldiminogruppen-haltigen Verbindungen **AV** sind, wie die Aldimine der Formel (I), unter geeigneten Bedingungen, insbesondere unter Ausschluss von Feuchtigkeit, lagerstabil. Heterofunktionelle Aldiminogruppen-haltige Verbindungen **AV,** die neben Aldiminogruppen zusätzliche für Polyreaktionen zugängliche Reaktivgruppen enthalten, sind dann lagerstabil, wenn sie von Reaktionen dieser Reaktivgruppen auslösenden Faktoren, wie beispielsweise Hitze oder UV-Strahlung, ferngehalten werden.

Bei Zutritt von Feuchtigkeit können die Aldiminogruppen der Aldiminogruppen-haltigen Verbindungen **AV** über Zwischenstufen formal zu Aminogruppen hydrolysieren, wobei der entsprechende, zur Herstellung des Aldimins verwendete Aldehyd **ALD** der Formel (IV) freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen teilweise oder vollständig hydrolysieren. Im speziellen Fall von heterofunktionellen Aldiminogruppen-haltigen Verbindungen **AV,** die gegenüber Aminen reaktive Gruppen, insbesondere Isocyanatgruppen, enthalten, reagieren die hydrolysierenden Aldiminogruppen hingegen weiter, beispielsweise mit Isocyanatgruppen zu Harnstoffgruppen. In diesem Fall kommt es zur Vernetzung der heterofunktionellen Aldiminogruppen-haltigen Verbindung **AV,** welche auch ohne Beteiligung weiterer Substanzen direkt zu einer ausgehärteten Polymerzusammensetzung führen kann. Die Reaktion der gegenüber Aminen reaktiven Gruppen mit den hydrolysierenden Aldiminogruppen muss dabei nicht notwendigerweise über Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer gegenüber Aminen reaktiven Gruppe reagiert.

Die Aldiminogruppen-haltigen Verbindungen **AV** lassen sich sehr breit verwenden. Grundsätzlich lassen sie sich überall dort einsetzen, wo sie als Quelle der Aldehyde **ALD** der Formel (IV) oder der aus der Aldiminogruppen-haltigen Verbindung **AV** bei der Hydrolyse entstehenden Amine der Formel (III) dienen können. Insbesondere können sie in der Funktion von geschützten Aminen, beziehungsweise geschützten Aldehyden, in Aldehyd- und/oder oder Amin-reaktiven Systemen eingesetzt werden und dort bei Bedarf gezielt entschützt werden. Insbesondere finden sie in Systemen Anwendungen, in welchen Verbindungen vorhanden sind, welche mit primären Aminen reagieren. Die Entschützung erfolgt hydrolytisch, beispielsweise durch Kontakt mit Wasser oder Feuchtigkeit, insbesondere Luftfeuchtigkeit.

Andererseits finden die Aldiminogruppen-haltigen Verbindungen **AV** Verwendung in Polymerzusammensetzungen enthaltend Reaktivgruppen, wie sie auch in der Verbindung **D** vorhanden sind.

Insbesondere geeignet sind die Aldiminogruppen-haltigen Verbindungen **AV** als Bestandteil von Isocyanatgruppen aufweisenden Polyurethanzusammensetzungen, welche unter Einwirkung von Feuchtigkeit vernetzen. Solche Polyurethanzusammensetzungen enthaltend Aldiminogruppen-haltige Verbindungen **AV** können ein- oder zweikomponentig sein. Sie sind geeignet als Klebstoffe, Dichtstoffe, Vergussmassen, Beschichtungen, Bodenbeläge, Anstriche, Lacke, Primer oder Schäume, sowie als rasch aushärtende reaktive Polyurethan-Heissschmelzklebstoffe, welche einen niedrigen Gehalt an Isocyanat-Monomeren aufweisen.

Eine bevorzugte Ausführungsform der Aldiminogruppen-haltige Verbindung **AV** ist die Aldiminogruppen-haltige Verbindung **AV1** der Formel (VI), 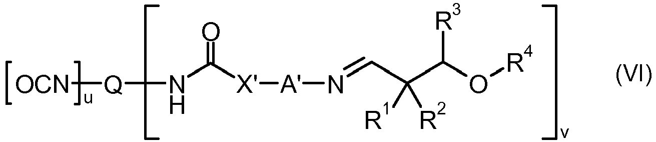 wobei
u für 0 oder 1 oder 2 oder 3 oder 4 oder 5 steht,
v für 1 oder 2 oder 3 oder 4 oder 5 oder 6 steht,
   mit der Massgabe, dass (u+v) für 2 oder 3 oder 4 oder 5 oder 6 steht;
Q für den Rest eines (u+v) Isocyanatgruppen aufweisenden Polyisocyanates nach Entfernung aller Isocyanatgruppen steht;
A' entweder
   für einen zweiwertigen, gegebenenfalls Heteroatome aufweisenden, Kohlenwasserstoffrest mit 2 bis 20 C-Atomen steht,
   oder zusammen mit R^{7'} für einen dreiwertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht; und
X' für O oder S oder N-R^{6'} oder N-R^{7'} steht,
   wobei R^{6'}
      entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
      oder für einen Substituenten der Formel (II') steht, 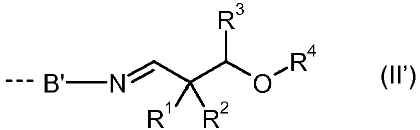 wobei B' für einen zweiwertigen, gegebenenfalls Heteroatome aufweisenden, Kohlenwasserstoffrest mit 2 bis 20 C-Atomen steht, und
   R^{7'} zusammen mit A' für einen dreiwertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht;
und R¹, R², R³ und R⁴ die bereits genannten Bedeutungen aufweisen.

Mit "Poly" beginnende Substanznamen wie Polyisocyanat oder Polyol bezeichnen im vorliegenden Dokument Substanzen, die formal zwei oder mehr der in ihrem Namen vorkommenden funktionellen Gruppen pro Molekül enthalten.

In der Formel (VI) stehen R¹ und R² bevorzugt für je eine Methylgruppe; R³ steht bevorzugt für ein Wasserstoffatom; R⁴ steht bevorzugt für einen Rest 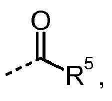 wobei R⁵ die bereits genannten Bedeutungen aufweist.

Eine Aldiminogruppen-haltige Verbindung **AV1** der Formel (VI) ist erhältlich durch die Umsetzung von mindestens einem Polyisocyanat der Formel (VII) mit mindestens einem nur einen aktiven Wasserstoff aufweisenden Aldimin der Formel (VIII).

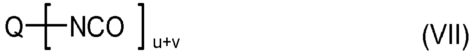

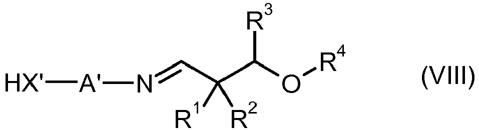

In den Formeln (VII) und (VIII) weisen Q, u, v, X', A', R¹, R², R³ und R⁴ die bereits beschriebenen Bedeutungen auf.

Ein geeignetes Polyisocyanat der Formel (VII) ist in einer Ausführungsform ein Isocyanatgruppen aufweisendes Polyurethanpolymer **PUP.**

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharn-stoffe, Polyester-Polyharnstoffe, Polyisocyanurate und Polycarbodiimide.

Ein geeignetes Isocyanatgruppen aufweisendes Polyurethanpolymer **PUP** ist erhältlich durch die Umsetzung von mindestens einem Polyol mit mindestens einem Polyisocyanat.

Als Polyole für die Herstellung eines Polyurethanpolymers **PUP** können beispielsweise die folgenden handelsüblichen Polyole oder Mischungen davon eingesetzt werden:
- Polyetherpolyole, auch Polyoxyalkylenpolyole oder Oligoetherole genannt, welche Polymerisationsprodukte von Ethylenoxid, 1,2-Propylenoxid, 1,2-oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Als Polyetherpolyole besonders geeignet sind Polyoxyalkylendiole und -triole, insbesondere Polyoxyalkylendiole. Besonders geeignete Polyoxyalkylendi- und triole sind Polyoxyethylendi- und -triole sowie Polyoxypropylendi- und -triole.

Besonders geeignet sind Polyoxypropylendiole und -triole mit einem Ungesättigtheitsgrad tiefer als 0.02 mEq/g und einem Molekulargewicht im Bereich von 1000 bis 30'000 g/mol, sowie Polyoxypropylendiole und -triole mit einem Molekulargewicht von 400 bis 8'000 g/mol. Unter 'Molekulargewicht' oder 'molgewicht' versteht man im vorliegenden Dokument stets das Molekulargewichtsmittel Mₙ. Insbesondere geeignet sind Polyoxypropylendiole mit einem Ungesättigtheitsgrad tiefer als 0.02 mEq/g und einem Molekulargewicht im Bereich von 1000 bis 12'000, insbesondere zwischen 1000 und 8000 g/mol. Solche Polyetherpolyole werden beispielsweise unter dem Handelsnamen Acclaim^{®} von Bayer vertrieben.

Ebenfalls besonders geeignet sind sogenannte "EO-endcapped" (ethylene oxide-endcapped) Polyoxypropylendiole und -triole. Letztere sind spezielle Polyoxypropylenpolyoxyethylenpolyole, die beispielsweise dadurch erhalten werden, dass reine Polyoxypropylenpolyole nach Abschluss der Polypropoxylierung mit Ethylenoxid alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.

Insbesondere geeignet sind Polyesterpolyole, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Caprolacton und Startern wie den vorgenannten zwei- oder dreiwertigen Alkoholen.

Besonders geeignete Polyesterpolyole sind Polyesterdiole. Insbesondere geeignete Polyesterdiole sind solche hergestellt aus Adipinsäure, Azelainsäure, Sebacinsäure, Dodecandicarbonsäure, Dimerfettsäure, Phthalsäure, Isophthalsäure und Terephthalsäure als Dicarbonsäure und aus Ethylenglykol, Diethylenglykol, Neopentylglykol, 1,4-Butandiol, 1,6-Hexandiol, Dimerfettsäurediol und 1,4-Cyclohexandimethanol als zweiwertigem Alkohol. Insbesondere geeignet sind auch Polyesterdiole hergestellt aus ε-Caprolacton und einem der vorgenannten zweiwertigen Alkohole als Starter.

Insbesondere geeignet sind bei Raumtemperatur flüssige, amorphe, teilkristalline und kristalline Polyesterdi- und -triole, insbesondere Polyesterdiole. Geeignete bei Raumtemperatur flüssige Polyesterpolyole sind nicht weit unterhalb von Raumtemperatur fest, beispielsweise bei Temperaturen zwischen 0 °C und 25 °C, und werden vorzugsweise in Kombination mit mindestens einem amorphen, teilkristallinen oder kristallinen Polyesterpolyol eingesetzt.
- Polycarbonatpolyole, wie sie durch Umsetzung beispielsweise der oben genannten - zum Aufbau der Polyesterpolyole eingesetzten - Alkohole mit Dialkylcarbonaten, wie Dimethylcarbonat, Diarylcarbonaten, wie Diphenylcarbonat, oder Phosgen zugänglich sind.
   Besonders geeignet sind Polycarbonatdiole, insbesondere amorphe Polycarbonatdiole.
- Als Polyole ebenfalls geeignet sind mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen.
- Polyacrylat- und Polymethacrylatpolyole.
- Polyhydroxyfunktionelle Fette und Öle, beispielsweise natürliche Fette und Öle, insbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Ethylen-Propylen-, Ethylen-Butylen- oder Ethylen-Propylen-Dien-Copolymere, wie sie beispielsweise von der Firma Kraton Polymers hergestellt werden, oder polyhydroxyfunktionelle Copolymere aus Dienen wie 1,3-Butandien oder Diengemischen und Vinylmonomeren wie Styrol, Acrylnitril oder Isobutylen, oder polyhydroxyfunktionelle Polybutadienpolyole, wie beispielsweise solche, die durch Copolymerisation von 1,3-Butadien und Allylalkohol hergestellt werden und auch hydriert sein können.
- Polyhydroxyfunktionelle Acrylonitril/Butadien-Copolymere, wie sie beispielsweise aus Epoxiden oder Aminoalkoholen und carboxylterminierten Acrylonitril/Butadien-Copolymeren (kommerziell erhältlich unter dem Namen Hycar^{®} CTBN von Hanse Chemie) hergestellt werden können.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, niedrigmolekulare Alkoxylierungsprodukte der vorgenannten zwei- und mehrwertigen Alkohole, sowie Mischungen der vorgenannten Alkohole bei der Herstellung eines Polyurethanpolymers **PUP** mitverwendet werden.

Als Polyisocyanate für die Herstellung eines Polyurethanpolymers **PUP** können handelsübliche aliphatische, cycloaliphatische oder aromatische Polyisocyanate, insbesondere Diisocyanate, verwendet werden, beispielsweise die folgenden:
1,6-Hexamethylendiisocyanat (HDI), 2-Methylpentamethylen-1,5-diisocyanat, 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat und beliebige Gemische dieser Isomeren, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), Oligomere und Polymere der vorgenannten Isocyanate, sowie beliebige Mischungen der vorgenannten Isocyanate. Bevorzugt sind monomere Diisocyanate, insbesondere MDI, TDI, HDI und IPDI.

Die Herstellung des Polyurethanpolymers **PUP** erfolgt in bekannter Art und Weise direkt aus den Polyisocyanaten und den Polyolen, oder durch schrittweise Adduktionsverfahren, wie sie auch als Kettenverlängerungsreaktionen bekannt sind.

In einer bevorzugten Ausführungsform wird das Polyurethanpolymer **PUP** über eine Reaktion von mindestens einem Polyisocyanat und mindestens einem Polyol hergestellt, wobei die Isocyanatgruppen gegenüber den Hydroxylgruppen im stöchiometrischen Überschuss vorliegen. Vorteilhaft beträgt das Verhältnis zwischen Isocyanat- und Hydroxylgruppen 1.3 bis 5, insbesondere 1.5 bis 3.

Vorteilhaft wird die Umsetzung bei einer Temperatur durchgeführt, bei der die verwendeten Polyole, Polyisocyanate und das gebildete Polyurethanpolymer flüssig vorliegen.

Das Polyurethanpolymer **PUP** weist ein Molekulargewicht von vorzugsweise über 500 g/mol, insbesondere ein solches zwischen 1000 und 50'000 g/mol, bevorzugt ein solches zwischen 2000 und 30'000 g/mol, auf.

Weiterhin weist das Polyurethanpolymer **PUP** bevorzugt eine mittlere Funktionalität im Bereich von 1.8 bis 3 auf.

In einer Ausführungsform ist das Polyurethanpolymer **PUP** ein bei Raumtemperatur festes Polyurethanpolymer **PUP1.** Ein bei Raumtemperatur festes Polyurethanpolymer **PUP1** ist vorteihaft erhältlich ausgehend von Polyetherpolyolen, Polyesterpolyolen Polycarbonatpolyolen. Insbesondere geeignet sind bei Raumtemperatur flüssige, amorphe, teilkristalline und kristalline Polyester- und Polycarbonatdi- und -triole, insbesondere Polyesterdiole und Polycarbonatdiole, wobei bei Raumtemperatur flüssige Polyester- und Polycarbonatdi- und -triole nicht weit unterhalb von Raumtemperatur fest sind, beispielsweise bei Temperaturen zwischen 0 °C und 25 °C, und vorzugsweise in Kombination mit mindestens einem amorphen, teilkristallinen oder kristallinen Polyol eingesetzt werden.

Die Polyester- und Polycarbonatdi- und -triole weisen vorteilhaft ein Molekulargewicht von 500 bis 5'000 g/mol auf.

Ein bei Raumtemperatur festes Polyurethanpolymer **PUP1** kann kristallin, teilkristallin oder amorph sein. Für ein teilkristallines oder amorphes Polyurethanpolymer **PUP1** gilt dabei, dass es bei Raumtemperatur nicht oder nur wenig fliessfähig ist, das heisst insbesondere, dass es bei 20 °C eine Viskosität von mehr als 5'000 Pa·s aufweist.

Das Polyurethanpolymer **PUP1** weist bevorzugt ein mittleres Molekulargewicht von 1'000 bis 10'000 g/mol, insbesondere von 2'000 bis 5'000 g/mol, auf

Ein zur Umsetzung mit einem Aldimin der Formel (VIII) geeignetes Polyisocyanat der Formel (VII) ist in einer weiteren Ausführungsform ein Polyisocyanat **PI** in der Form eines Diisocyanates oder eines niedrigmolekularen Oligomeren eines Diisocyanates oder eines Derivates eines Diisocyanates, wobei als Diisocyanat dieselben Diisocyanate geeignet sind, welche bereits als geeignet zur Herstellung eines Polyurethanpolymers **PUP** genannt wurden.

Als Polyisocyanat **PI** eignen sich vor allem Oligomere oder Oligomerengemische von Diisocyanaten, insbesondere von HDI, IPDI, TDI und MDI. Kommerziell erhältliche Typen sind insbesondere HDI-Biurete, beispielsweise als Desmodur^{®} N 100 und N 3200 (Bayer), Tolonate^{®} HDB und HDB-LV (Rhodia) und Duranate^{®} 24A-100 (Asahi Kasei); HDI-Isocyanurate, beispielsweise als Desmodur^{®} N 3300, N 3600 und N 3790 BA (alle von Bayer), Tolonate^{®} HDT, HDT-LV und HDT-LV2 (Rhodia), Duranate^{®} TPA-100 und THA-100 (Asahi Kasei) und Coronate^{®} HX (Nippon Polyurethane); HDI-Uretdione, beispielsweise als Desmodur^{®} N 3400 (Bayer); HDI-Iminooxadiazindione, beispielsweise als Desmodur^{®} XP 2410 (Bayer); HDI-Allophanate, beispielsweise als Desmodur^{®} VP LS 2102 (Bayer); IPDI-Isocyanurate, beispielsweise in Lösung als Desmodur^{®} Z 4470 (Bayer) oder in fester Form als Vestanat^{®} T1890/100 (Degussa); TDI-Oligomere, beispielsweise als Desmodur^{®} IL (Bayer); gemischte Isocyanurate auf Basis TDI / HDI, zum Beispiel als Desmodur^{®} HL (Bayer).

Als Polyisocyanat **PI** bevorzugt sind die Oligomere von HDI und/oder IPDI, insbesondere die Isocyanurate.

Die vorgenannten Polyisocyanate **PI** stellen üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid , Uretonimin- oder Oxadiazintrion-Gruppen.

Ebenfalls möglich ist, dass ein zur Umsetzung mit einem Aldimin der Formel (VIII) geeignetes Polyisocyanat der Formel (VII) eine Mischung bestehend aus mindestens einem Polyurethanpolymer **PUP** und mindestens einem Polyisocyanat **PI** ist.

Ein zur Umsetzung mit einem Polyisocyanat der Formel (VII) geeignetes Aldimin der Formel (VIII) weist nur einen aktiven Wasserstoff auf. Ein solches Aldimin der Formel (VIII) ist grundsätzlich auf die gleiche Weise erhältlich wie bereits vorgängig für ein Aldimin der Formel (I) beschrieben, mit der Einschränkung, dass nicht alle Amine **B1** der Formel (III), wie sie vorgängig genannt wurden, geeignet sind. Zur Herstellung eines Aldimins der Formel (VIII) sind in einer Ausführungsform Amine C der Formel (IX) geeignet,

HX^{a'}-A'-NH₂ (IX)

wobei
X^{a'} für O oder S oder N-R^{6a'} oder N-R^{7'} steht,
   wobei R^{6a'}
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (X) steht,

   --- B'-NH₂ (X)
und A', B' und R^{7'} die bereits genannten Bedeutungen aufweisen.

Mindestens ein Amin **C** der Formel (IX) kann mit mindestens einem der bereits vorgängig genannten Aldehyde **ALD** der Formel (IV) in der bereits beschriebenen Weise zu Aldiminen der Formel (VIII) umgesetzt werden.

Geeignete Amine **C** der Formel (IX) sind in einer Ausführungsform Verbindungen mit einer oder zwei primären aliphatischen und einer sekundären Aminogruppe, wie sie bereits als geeignete Amine **B1** erwähnt wurden. In einer weiteren Ausführungsform sind geeignete Amine **C** der Formel (IX) aliphatische Hydroxyamine oder aliphatische Mercaptoamine, welche eine oder mehrere primäre Aminogruppen tragen, wie sie bereits als geeignete Amine **B1** erwähnt wurden.

Als Amin **C** bevorzugt sind N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin; Produkte aus der Michael-artigen Addition von aliphatischen primären Diaminen mit Malein- und Fumarsäurediestern, Acryl- und Methacrylsäureestern, Acryl- und Methacrylsäureamiden, bevorzugt mit Maleinsäurediestern, insbesondere Maleinsäuredimethyl-, -diethyl-, -dipropyl- und -dibutylester, und mit Acrylsäureestern, insbesondere Acrylsäuremethylester, umgesetzt im Molverhältnis 1:1; aliphatische Hydroxy- oder Mercaptoamine, in denen die primäre Aminogruppe von der Hydroxyl- oder Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxyethoxy)-propylamin, 3-(2-(2-Hydroxyethoxy)-ethoxy)-propylamin sowie 3-(6-Hydroxyhexyloxy)-propylamin.

Als Amin **C** besonders bevorzugt sind N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT, Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin sowie 3-(6-Hydroxyhexyloxy)-propylamin.

In einer Ausführungsform des Aldimins der Formel (VIII) steht X' für N-R^{6'}. Derartige Aldimine der Formel (VIII) lassen sich gegebenenfalls auf einem leicht anderern Syntheseweg als dem bisher beschriebenen herstellen. Dieser Syntheseweg besteht darin, dass ein Aldehyd **ALD** der Formel (IV) mit einem zweiwertigen aliphatischen primären Amin **B2** der Formel H₂N-A^{a}-NH₂ - wobei A^{a} für einen zweiwertigen, gegebenenfalls Heteroatome aufweisenden, Kohlenwasserstoffrest mit 2 bis 20 C-Atomen steht - in einem ersten Schritt zu einem Zwischenprodukt umgesetzt wird, welches neben einer Aldiminogruppe noch eine primäre Aminogruppe enthält. Dieses Zwischenprodukt wird anschliessend in einem zweiten Schritt zu einem Aldimin der Formel (VIII) umgesetzt, indem die primäre Aminogruppe mit Hilfe eines Michael-Akzeptors einfach alkyliert wird. Diese Umsetzung erfolgt dabei in der gleichen Weise wie bereits vorgängig für das Aldimin der Formel (I) beschrieben, wobei dieselben Amine **B2** und dieselben Michael-Akzeptoren geeignet sind.

Die Umsetzung von mindestens einem Polyisocyanat der Formel (VII) mit mindestens einem Aldimin der Formel (VIII) zu mindestens einer Aldiminogruppen-haltigen Verbindung **AV1** der Formel (VI) erfolgt unter bekannten Bedingungen, wie sie für Reaktionen zwischen den an der jeweiligen Umsetzung beteiligten Reaktivgruppen typischerweise verwendet werden und wie sie bereits vorgängig erwähnt wurden.

Wird diese Additionsreaktion stöchiometrisch durchgeführt, das heisst mit einem Molequivalent aktivem Wasserstoff des Aldimins der Formel (VIII) auf ein Molequivalent Isocyanatgruppen des Polyisocyanats der Formel (VII) - wodurch dessen Reaktivgruppen vollständig umgesetzt werden -, wird als Additionsprodukt eine Aldiminogruppen-haltige Verbindung **AV1** der Formel (VI) mit dem Index u gleich null erhalten. Eine solche Verbindung **AV1** ist ein Polyaldimin.

Wird diese Additionsreaktion hingegen unterstöchiometrisch geführt, das heisst mit weniger als einem Molequivalent aktivem Wasserstoff des Aldimins der Formel (VIII) auf ein Molequivalent Isocyanatgruppen des Polyisocyanats der Formel (VII) - wodurch die Isocyanatgruppen nur teilweise umgesetzt werden -, wird als Additionsprodukt eine heterofunktionelle Verbindung erhalten, das heisst eine Aldiminogruppen-haltige Verbindung **AV1** der Formel (VI), die neben einer oder mehreren Aldiminogruppen noch mindestens eine Isocyanatgruppe aufweist. Der Index u in Formel (VI) ist in diesem Fall grösser als null.

Die Aldiminogruppen-haltigen Verbindungen **AV1** der Formel (VI) sind unter geeigneten Bedingungen, insbesondere unter Ausschluss von Feuchtigkeit, lagerstabil.

Ein weiterer Gegenstand der vorliegenden Erfindung ist eine Zusammensetzung, umfassend
a) mindestens eine Aldiminogruppen-haltige Verbindung **AV1** der Formel (VI); und
b) gegebenenfalls mindestens ein Polyisocyanat **P;**
mit der Massgabe, dass das Verhältnis der Anzahl Aldiminogruppen zu der Anzahl Isocyanatgruppen in der Zusammensetzung höchstens 1 beträgt.

Als Aldiminogruppen-haltige Verbindungen **AV1** der Formel (VI) sind die vorgängig beschriebenen geeignet, beziehungsweise die bevorzugten Ausführungsformen davon, wie sie vorgängig beschrieben wurden.

Die Zusammensetzung umfasst neben mindestens einer Aldiminogruppen-haltigen Verbindung **AV1** der Formel (VI) gegebenenfalls mindestens ein Polyisocyanat **P,** mit der Massgabe, dass das Verhältnis der Anzahl Aldiminogruppen zu der Anzahl Isocyanatgruppen in der Zusammensetzung höchstens 1 beträgt.

Für den Fall, dass die in der Zusammensetzung enthaltenen Aldiminogruppen-haltigen Verbindungen **AV1** der Formel (VI) im Durchschnitt einen Index u grösser oder gleich dem Index v aufweisen, ist die Verbindung **AV1** der Formel (VI) mittels Feuchtigkeit selbsthärtend. Das heisst, durch die Einwirkung von Feuchtigkeit kann eine solche Verbindung **AV1** der Formel (VI) auch allein zu einem hochmolekularen Polyurethan-Kunststoff vernetzen. Die Anwesenheit eines Polyisocyanates **P** ist in diesem Fall optional.

Für den Fall, dass die in der Zusammensetzung enthaltenen Aldiminogruppen-haltigen Verbindungen **AV1** der Formel (VI) im Durchschnitt einen Index u kleiner als den Index v aufweisen, so muss mindestens ein Polyisocyanat **P** in der Zusammensetzung vorhanden sein, und zwar mindestens in einer solchen Menge, dass pro in der Zusammensetzung vorhandene Aldiminogruppe mindestens eine Isocyanatgruppe vorhanden ist, sodass durch die Einwirkung von Feuchtigkeit ein hochmolekularer Polyurethan-Kunststoff gebildet wird.

Als Polyisocyanat **P** geeignet ist in einer Ausführungsform ein Isocyanatgruppen aufweisendes Polyurethanpolymer **PUP,** wie es bereits vorgängig als geeignetes Polyisocyanat der Formel (VII) zur Herstellung der Aldiminogruppen-haltigen Verbindung **AV1** der Formel (VI) beschrieben wurde.

Als Polyisocyanat **P** geeignet ist in einer weiteren Ausführungsform ein Polyisocyanat **PI** in der Form eines Diisocyanates oder eines niedrigmolekularen Oligomeren eines Diisocyanates oder eines Derivates eines Diisocyanates, wie es ebenfalls vorgängig als geeignetes Polyisocyanat der Formel (VII) zur Herstellung der Aldiminogruppen-haltigen Verbindung **AV1** der Formel (VI) beschrieben wurde.

Ebenfalls möglich als Polyisocyanat **P** ist eine Mischung bestehend aus mindestens einem Polyurethanpolymer **PUP** und mindestens einem Polyisocyanat **PI.**

Bevorzugt handelt es sich beim in der Zusammensetzung gegebenenfalls vorhandenen Polyisocyanat **P** um das gleiche Polyisocyanat wie das der Aldiminogruppen-haltigen Verbindung **AV1** der Formel (VI) zugrunde liegende Polyisocyanat der Formel (VII).

Bevorzugt ist eine Zusammensetzung umfassend sowohl eine Aldiminogruppen-haltigeVerbindung **AV1** der Formel (VI) als auch ein Polyisocyanat **P** erhältlich, indem mindestens ein Polyisocyanat der Formel (VII) mit mindestens einem Aldimin der Formel (VIII) in einem solchen Verhältnis umgesetzt wird, dass nach der Umsetzung ein Restgehalt an nicht umgesetztem Polyisocyanat der Formel (VII) als Polyisocyanat **P** in der Zusammensetzung verbleibt.

Es ist aber auch möglich, bei der Herstellung einer Aldiminogruppenhaltigen Verbindung **AV1** das dazu verwendete Polyisocyanat der Formel (VII) vollständig umzusetzen und anschliessend gegebenenfalls ein Polyisocyanat **P** zur Verbindung **AV1** zu geben.

Zusammensetzungen umfassend mindestens eine Aldiminogruppen-haltige Verbindung **AV1** der Formel (VI) und gegebenenfalls mindestens ein Polyisocyanat **P** weisen einen überraschend niedrigen Gehalt an monomeren Diisocyanaten auf, sofern das der Verbindung **AV1** zugrunde liegende Polyisocyanat der Formel (VII) und das Polyisocyanat **P** entweder ein Polyurethanpolymer **PUP** oder ein Polyisocyanat **PI** in der Form eines oligomeren Diisocyanates ist.

In einem weiteren Aspekt betrifft die Erfindung ein Verfahren zur Verminderung des Gehalts an monomeren Diisocyanaten in Isocyanatgruppen aufweisenden Polyurethanpolymeren oder in oligomeren Polyisocyanaten, oder in Zusammensetzungen, welche Isocyanatgruppen aufweisende Polyurethanpolymere oder oligomere Polyisocyanate enthalten, in dem diese mit mindestens einem Aldimin der Formel (I), wie vorgängig beschrieben, umgesetzt werden.

Bei der Umsetzung von Polyolen mit Diisocyanaten zu Isocyanatgruppen aufweisenden Polyurethanpolymeren **PUP** verbleibt aufgrund der statistischen Verteilung der möglichen Reaktionsprodukte ein Restgehalt an nicht umgesetzten monomeren Diisocyanaten im gebildeten Polymer. Ebenso verbleibt bei der Herstellung von oligomeren Polyisocyanaten ein Restgehalt an nicht umgesetzten monomeren Diisocyanaten im Produkt. Diese monomeren Diisocyanate, auch kurz "Isocyanat-Monomere" genannt, sind flüchtige Verbindungen und können aufgrund ihrer reizenden, allergenen und/oder toxischen Wirkung schädlich sein. In vielen Anwendungsgebieten sind sie deshalb unerwünscht. Dies gilt insbesondere für Spritzapplikationen sowie für heiss zu verarbeitende Zusammensetzungen, wie beispielsweise Heissschmelzklebstoffe.

Die unvollständige Umsetzung eines Polyisocyanats der Formel (VII), welches einen bestimmten Gehalt an monomerem Diisocyanat aufweist, mit einem Aldimin der Formel (VIII) in der vorgängig beschriebenen Weise führt zu einer Zusammensetzung, die einen überraschend niedrigen Gehalt an monomeren Diisocyanaten aufweist; dieser liegt deutlich tiefer als jener des Polyisocyanats der Formel (VII) vor der Umsetzung.

Der überraschend niedrige Gehalt an monomeren Diisocyanaten wird vermutlich dadurch erreicht, dass bei der beschriebenen Umsetzung mindestens eines Polyisocyanats der Formel (VII) mit mindestens einem Aldimin der Formel (VIII) zu mindestens einer Aldiminogruppen-haltigen Verbindung **AV1** der Formel (VI) der aktive Wasserstoff des Aldimins der Formel (VIII) bevorzugt mit den im Polyisocyanat vorhandenen monomeren Diisocyanaten reagiert. Auf diese Weise wird ein grosser Teil der ursprünglich vorhandenen Diisocyanat-Monomere umgesetzt, auch wenn die Umsetzung mit einer solchen Stöchiometrie durchgeführt wird, dass ein Anteil des Polyisocyanats der Formel (VII) nicht mit dem Aldimin der Formel (VIII) reagiert, sondern als Polyisocyanat **P** in der Zusammensetzung verbleibt. Auf diese Weise kann der Gehalt an monomeren Diisocyanaten einer Zusammensetzung stark reduziert werden.

Bevorzugt weist eine solche Zusammensetzung einen Gehalt an monomeren Diisocyanaten ≤ 1 Gewichts-%, insbesondere ≤ 0.5 Gewichts-%, bezogen auf die feuchtigkeitsreaktiven Bestandteile der Zusammensetzung auf.

Zusammensetzungen umfassend mindestens eine Aldiminogruppenhaltige Verbindung **AV1** der Formel (VI) und gegebenenfalls mindestens ein Polyisocyanat **P** können gegebenenfalls weitere Hilfs- und Zusatzstoffe enthalten. Dafür sind beispielsweise die folgenden Substanzen geeignet:
- Weichmacher, beispielsweise Carbonsäureester wie Phthalate, beispielsweise Dioctylphthalat, Diisononylphthalat oder Diisodecylphthalat, Adipate, beispielsweise Dioctyladipat, Azelate und Sebacate, organische Phosphor- und Sulfonsäureester oder Polybutene;
- Lösemittel;
- anorganische und organische Füllstoffe, zum Beispiel gemahlene oder gefällte Calciumcarbonate, welche gegebenenfalls mit Stearaten beschichtet sind, Russe, insbesondere industriell hergestellte Russe (im Folgenden als "Russ" bezeichnet), Baryt (BaSO4, auch Schwerspat genannt), Kaoline, Aluminiumoxide, Aluminiumhydroxide, Kieselsäuren, insbesondere hochdisperse Kieselsäuren aus Pyrolyseprozessen, PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse der Aldimine beschleunigen, wie beispielsweise organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen beschleunigen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismuttris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel, zum Beispiel Harnstoffverbindungen, Polyamidwachse, Bentonite oder pyrogene Kieselsäuren;
- Reaktivverdünner und Vernetzer, beispielsweise monomere Polyisocyanate wie MDI, TDI, Gemische aus MDI und MDI-Homologen (polymeres MDI oder PMDI), sowie Oligomere dieser Polyisocyanate, insbesondere in Form von Isocyanuraten, Carbodiimiden, Uretoniminen, Biureten, Allophanaten oder Iminooxadiazindionen, Addukte monomerer Polyisocyanate mit kurzkettigen Polyolen, sowie Adipinsäuredihydrazid und andere Dihydrazide;
- weitere latente Härter mit geschützten Aminogruppen, wie beispielsweise Ketimine, Oxazolidine, Enamine oder andere Aldimine;
- Trocknungsmittel, wie beispielsweise Molekularsiebe, Calciumoxid, hochreaktive Isocyanate wie p-Tosylisocyanat, Orthoameisensäureester, Alkoxysilane wie Tetraethoxysilan, Organoalkoxysilane wie Vinyltrimethoxysilan, und Organoalkoxysilane, welche in α-Stellung zur Silangruppe eine funktionelle Gruppe aufweisen;
- Haftvermittler, insbesondere Organoalkoxysilane ("Silane") wie beispielsweise Epoxysilane, Vinylsilane, (Meth)acrylsilane, Isocyanatosilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, darauf zu achten, dass solche Zusätze die Lagerstabilität der Zusammensetzung nicht beeinträchtigen. Das heisst, dass diese Zusätze während der Lagerung die zur Vernetzung führenden Reaktionen, wie Hydrolyse der Aldiminogruppen oder Vernetzung der Isocyanatgruppen, nicht in signifikantem Ausmass auslösen dürfen. Insbesondere bedeutet dies, dass all diese Zusätze kein oder höchstens Spuren von Wasser enthalten sollten. Es kann sinnvoll sein, gewisse Zusätze vor dem Einmischen in die Zusammensetzung chemisch oder physikalisch zu trocknen.

Solche Zusätze können der Zusammensetzung nach erfolgter Herstellung der Verbindung **AV1** der Formel (VI) zugegeben werden. Sie können aber auch bereits bei der Umsetzung von mindestens einem Polyisocyanat der Formel (VII) mit mindestens einem Aldimin der Formel (VIII) zu mindestens einer Verbindung **AV1** der Formel (VI) vorhanden sein.

Bevorzugt enthält die beschriebene Zusammensetzung neben mindestens einer Verbindung **AV1** der Formel (VI) und gegebenenfalls mindestens einem Polyisocyanat **P** mindestens einen Katalysator Der Katalysator ist insbesondere eine der genannten Säuren, wie Benzoesäure oder Salicylsäure, oder eine der genannten Metallverbindungen, oder eine der genannten tertiären Amine. Insbesondere handelt es sich bei diesem Katalysator um einen Katalysator, welcher die Hydrolyse der Aldiminogruppen beschleunigt, bevorzugt eine Säure. Es kann durchaus auch vorteilhaft sein, wenn unterschiedliche Katalysatoren, bzw. unterschiedliche Katalysatorenarten, miteinander gemischt werden.

Die Zusammensetzung umfassend mindestens eine Aldiminogruppenhaltige Verbindung **AV1** der Formel (VI) und gegebenenfalls mindestens ein Polyisocyanat **P** wird unter Ausschluss von Feuchtigkeit aufbewahrt. Sie ist lagerstabil, d.h. sie kann unter Ausschluss von Feuchtigkeit in einer geeigneten Verpackung oder Anordnung, wie beispielsweise einem Fass, einem Hobbock, einem Eimer, einem Beutel, einer Kartusche oder einer Flasche über einen Zeitraum von mehreren Monaten bis zu einem Jahr und länger aufbewahrt werden, ohne dass sie sich in ihren Anwendungseigenschaften oder in ihren Eigenschaften nach der Aushärtung in einem für ihren Gebrauch relevanten Ausmass verändert. Üblicherweise wird die Lagerstabilität über die Messung der Viskosität ermittelt.

Die Aldiminogruppen der Verbindung **AV1** der Formel (VI) und gegebenenfalls vorhandene weitere Aldiminogruppen haben die Eigenschaft, bei Kontakt mit Feuchtigkeit zu hydrolysieren. Die in der Zusammensetzung vorhandenen Isocyanatgruppen reagieren formal mit den bei der Hydrolyse der Aldiminogruppen frei werdenden Aminogruppen, wobei der entsprechende Aldehyd **ALD** der Formel (IV) freigesetzt wird. Im Verhältnis zu den Aldiminogruppen überschüssige Isocyanatgruppen reagieren mit vorhandenem Wasser. Als Ergebnis dieser Reaktionen härtet die Zusammensetzung zu einem hochmolekularen Kunststoff aus; dieser Prozess wird auch als Vernetzung bezeichnet. Die Reaktion der Isocyanatgruppen mit den hydrolysierenden Aldiminogruppen muss dabei nicht notwendigerweise über die Aminogruppen erfolgen. Selbstverständlich sind auch Reaktionen mit Zwischenstufen der Hydrolyse der Aldiminogruppen zu Aminogruppen möglich. Beispielsweise ist es denkbar, dass hydrolysierende Aldiminogruppen in der Form von Halbaminalen direkt mit den Isocyanatgruppen reagieren. Im Fall einer Heissschmelzklebstoff-Zusammensetzung ist damit die Vernetzung gemeint, wodurch die Zusammensetzung nicht mehr aufschmelzbar ist. Die bei der Aushärtung freiwerdenden Aldehyde **ALD** weisen gegenüber im Stand der Technik üblicherweise zur Herstellung von Aldiminen eingesetzten Aldehyden, wie zum Beispiel Isobutyraldehyd oder Pivalaldehyd, einen geringeren Geruch auf, was für viele Anwendungen ein grosser Vorteil ist.

Das für die Aushärtungsreaktion benötigte Wasser kann entweder aus der Luft stammen (Luftfeuchtigkeit), oder aber die Zusammensetzung kann mit einer Wasser enthaltenden Komponente in Kontakt gebracht werden, zum Beispiel durch Besprühen, oder es kann der Zusammensetzung bei der Applikation eine Wasser enthaltende Komponente zugesetzt werden.

Die Zusammensetzung härtet bei Kontakt mit Feuchtigkeit im Allgemeinen ohne die Bildung von Blasen aus. Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge zugesetzten Wassers beeinflussen.

Falls die Zusammensetzung als Klebstoff für elastische Verklebungen verwendet wird, weist sie bevorzugt eine pastöse Konsistenz mit strukturviskosen Eigenschaften auf. Ein solcher Klebstoff wird mittels einer geeigneten Vorrichtung auf das Substrat aufgetragen, bevorzugt in Form einer Raupe, wobei diese eine im Wesentlichen runde oder dreieckige Querschnittsfläche aufweisen kann. Geeignete Methoden zum Auftragen des Klebstoffes sind beispielsweise die Applikation aus handelsüblichen Kartuschen, welche manuell oder mittels Druckluft betrieben werden, oder aus einem Fass oder Hobbock mittels einer Förderpumpe oder eines Extruders, gegebenenfalls mittels eines Applikationsroboters. Ein Klebstoff mit guten Applikationseigenschaften weist eine hohe Standfestigkeit und einen kurzen Fadenzug auf. Das heisst, er bleibt nach der Applikation in der applizierten Form stehen, fliesst also nicht auseinander, und zieht nach dem Absetzen des Applikationsgerätes keinen oder nur einen sehr kurzen Faden, so dass das Substrat nicht verschmutzt wird.

Die Zusammensetzung vernetzt mit wenig Wasser rasch und ohne die Bildung von Blasen. Für eine Anwendung beispielsweise als Klebstoff bedeutet dies, dass eine Klebeverbindung schon früh bis zu einem gewissen Grade belastbar ist, auch wenn erst relativ wenig Wasser aus der Umgebung mit der Zusammensetzung in Kontakt gekommen ist. Dies ist in der industriellen Fertigung, beispielsweise in der Montage von Fahrzeugen, ein grosser Vorteil, da angeklebte Bauteile schon nach relativ kurzer Zeit durch die Verklebung in Position gehalten werden sollen, und das geklebte Objekt dadurch ohne weitere Fixierung bewegt und weiter bearbeitet werden kann.

Die Zusammensetzung zeichnet sich im ausgehärteten Zustand über ausgezeichnete Eigenschaften aus. Sie verfügt beispielsweise über eine hohe Dehnbarkeit und eine hohe Zugfestigkeit. Ihr Elastizitätsmodul variiert in Abhängigkeit der zur Herstellung der Zusammensetzung verwendeten Komponenten, wie beispielsweise der Polyole, Polyisocyanate oder Diamine, und lässt sich an die Anforderungen einer bestimmten Applikation anpassen, beispielsweise auf hohe Werte für Klebstoffe oder auf tiefe Werte für Dichtstoffe.

Die Zusammensetzung kann für verschiedenste Zwecke verwendet werden. Beispielsweise ist sie geeignet als Klebstoff für das Verkleben von diversen Substraten, beispielsweise zum Verkleben von Bauteilen bei der Herstellung von Automobilen, Schienenfahrzeugen, Schiffen oder anderen industriellen Gütern, insbesondere als reaktiver Heissschmelzklebstoff, als Dichtstoff aller Art, beispielsweise zum Abdichten von Fugen im Bau, sowie als Beschichtung oder Belag für diverse Artikel beziehungsweise variable Substrate. Als Beschichtungen bevorzugt sind Schutzanstriche, Versiegelungen und Schutzbeschichtungen, sowie insbesondere Primer.

Unter einem "Primer" wird im vorliegenden Dokument eine als Voranstrich geeignete Zusammensetzung verstanden, die neben nichtreaktiven flüchtigen Stoffen und optional festen Zuschlägen mindestens ein Polymer und/oder mindestens eine Substanz mit reaktiven Gruppen enthält und die befähigt ist, bei der Applikation auf einem Substrat zu einem festen, gut haftenden Film in einer Schichtdicke von typischerweise mindestens 5 µm auszuhärten, wobei die Aushärtung entweder allein durch das Verdampfen der nichtreaktiven flüchtigen Stoffe, wie beispielsweise Lösemittel oder Wasser, oder durch eine chemische Reaktion, oder durch eine Kombination dieser Faktoren, zustande kommt, und welche eine gute Haftung zu einer nachfolgend aufgebrachten Schicht, beispielsweise einem Kleb- oder Dichtstoff, aufbaut.

Besonders geeignet sind die beschriebenen Zusammensetzungen für Anwendungen, in welchen ein niedriger Gehalt an monomeren Diisocyanaten gefordert ist. Dies sind insbesondere Anwendungen, bei welcher die Zusammensetzung gespritzt wird, und Anwendungen, bei welcher die Zusammensetzung bei erhöhter Temperatur appliziert wird, beispielsweise als Heissschmelzklebstoff.

In einer bevorzugten Ausführungsform ist die Zusammensetzung umfassend mindestens eine Aldiminogruppen-haltige Verbindung **AV1** der Formel (VI) und gegebenenfalls mindestens ein Polyisocyanat **P** ein reaktiver Heissschmelzklebstoff. In diesem Fall ist sowohl das zur Herstellung der Verbindung **AV1** der Formel (VI) verwendete Polyisocyanat der Formel (VII) als auch das gegebenenfalls vorhandene Polyisocyanat **P** ein bei Raumtemperatur festes, Isocyanatgruppen aufweisendes Polyurethanpolymer **PUP1,** wie es vorgängig beschrieben wurde.

Zusätzlich zu den bereits genannten Hilfs- und Zusatzstoffen kann ein solcher reaktiver Heissschmelzklebstoff gegebenenfalls mindestens ein nichtreaktives thermoplastisches Polymer enthalten, wobei beispielsweise Homo- oder Copolymere von ungesättigten Monomeren, insbesondere aus der Gruppe umfassend Ethylen, Propylen, Butylen, Isobutylen, Isopren, Vinylacetat oder höhere Ester davon, und (Meth)acrylat, wobei EthylenvinylacetatCopolymere (EVA), ataktische Poly-α-Olefine (APAO), Polypropylene (PP) und Polyethylene (PE) besonders geeignet sind.

Für die Wirkungsweise eines reaktiven Heissschmelzklebstoffes ist es wichtig, dass der Klebstoff aufschmelzbar ist, das heisst, dass er bei der Applikationstemperatur eine genügend niedrige Viskosität aufweist, um applizierbar zu sein, und dass er beim Erkalten möglichst schnell eine genügende Klebefestigkeit aufbaut, schon bevor die Vernetzungsreaktion mit Luftfeuchtigkeit abgeschlossen ist (Anfangsfestigkeit). Es hat sich gezeigt, dass die beschriebenen Zusammensetzungen, wenn sie ausgehend von Polyurethanpolymeren **PUP1** hergestellt sind, bei den für Heissschmelzklebstoffe üblichen Applikationstemperaturen im Bereich von 85 °C bis 200 °C, typischerweise 120 °C bis 160 °C, eine gut handhabbare Viskosität aufweisen, und dass sie beim Erkalten genügend schnell eine gute Klebefestigkeit aufbauen.

Bei der Applikation kommt der reaktive Heissschmelzklebstoff in Kontakt mit Feuchtigkeit, insbesondere in Form von Luftfeuchtigkeit. Parallel zur physikalischen Erhärtung infolge Erstarren beim Abkühlen setzt auch die chemische Vernetzung mit Feuchtigkeit ein, hauptsächlich indem die vorhandenen Aldiminogruppen durch Feuchtigkeit hydrolysiert werden und in der bereits beschriebenen Weise schnell mit den Isocyanatgruppen reagieren. Überschüssige Isocyanatgruppen vernetzen ebenfalls mit Feuchtigkeit in bekannter Weise.

Der beschriebene Heissschmelzklebstoff weist insbesondere einen niedrigen Gehalt an monomeren Diisocyanaten auf.

Der beschriebene Heissschmelz-Klebstoff zeigt bei der Vernetzung mit Feuchtigkeit eine stark verminderte Tendenz zur Bildung von Blasen, da bei der Vernetzung durch das Vorhandensein von Aldiminogruppen - je nach Stöchiometrie - wenig oder gar kein Kohlendioxid gebildet wird.

Die Dicke der Klebstoffschicht (Verklebungsdicke) bei einer Verklebung mittels Heissschmelz-Klebstoff beträgt typischerweise 10 Mikrometer oder mehr. Insbesondere liegt die Verklebungsdicke zwischen 10 Mikrometern und 20 Millimetern, vor allem zwischen 80 Mikrometern und 500 Mikrometern. Bei dicken Schichten ist die Vernetzung, bedingt durch die langsame Wasserdiffusion, jedoch üblicherweise sehr langsam.

Der beschriebene Heissschmelz-Klebstoff wird insbesondere in einem industriellen Fertigungsprozess eingesetzt.

In einer weiteren bevorzugten Ausführungsform ist die Zusammensetzung umfassend mindestens eine Aldiminogruppen-haltige Verbindung **AV1** der Formel (VI) und gegebenenfalls mindestens ein Polyisocyanat **P** eine Beschichtung, beispielsweise geeignet als Anstrich, Lack oder Primer.

In diesem Fall ist entweder das zur Herstellung der Verbindung **AV1** der Formel (VI) verwendete Polyisocyanat der Formel (VII), oder das gegebenenfalls vorhandene Polyisocyanat **P,** ein Polyisocyanat **PI,** wie es vorgängig beschrieben wurde, oder eine Mischung bestehend aus mindestens einem Polyurethanpolymer **PUP** und mindestens einem Polyisocyanat **PI.**

Als Hilfs- und Zusatzstoff enthält eine solche Beschichtung vorteilhaft mindestens ein Lösemittel. Geeignete Lösemittel sind beispielsweise Ketone wie Aceton, Methylethylketon, Methylisobutylketon, Diisobutylketon und Mesityloxid, sowie cyclische Ketone wie Cyclohexanon und Methylcyclohexanon; Ester wie Methylacetat, Ethylacetat, Propylacetat, Butylacetat, tert.Butylacetat, Formiate, Propionate oder Malonate; Ether wie Ketonether, Esterether und Dialkylether wie Diisopropylether, Diethylether, Dibutylether, Methyl-tert.butylether, Diethylenglykoldiethylether sowie Ethylenglykoldiethylether; aliphatische und aromatische Kohlenwasserstoffe wie Toluol, Xylol, Heptan, Octan, und Erdölfraktionen wie Naphtha, White Spirit, Petrolether oder Benzin; halogenierte Kohlenwasserstoffe wie Methylenchlorid; sowie N-alkylierte Lactame wie beispielsweise N-Methylpyrrolidon, N-Cyclohexylpyrrolidon oder N-Dodecylpyrrolidon.

Neben den bereits erwähnten Hilfs- und Zusatzstoffen eignen sich als Bestandteil der beschriebenen Beschichtung weiterhin Triisocyanate wie Tris-(4-isocyanatophenyl)-methan und Tris-(4-isocyanatophenyl)-thiophosphat; Titanate, bevorzugt solche, welche mindestens einen über eine Sauerstoff-Titan-Bindung an das Titanatom gebundenen Substituenten aufweisen, insbesondere eine Alkoxygruppe, Sulfonatgruppe, Carboxylatgruppe, Dialkylphosphatgruppe, Dialkylpyrophosphatgruppe oder eine Acetylacetonatgruppe, wobei bei mehreren Substituenten diese alle gleich oder untereinander gemischt sein können. Beispiele für geeignete Titanate sind die von Kenrich Petrochemicals unter dem Handelsnamen Ken-React^{®} erhältlichen Typen KR TTS, KR 7, KR 9S, KR 12, KR 26S, KR 33DS, KR 38S, KR 39DS, KR44, KR 134S, KR 138S, KR 158FS, KR212, KR 238S, KR 262ES, KR 138D, KR 158D, KR238T, KR 238M, KR238A, KR238J, KR262A, LICA 38J, KR 55, LICA 01, LICA 09, LICA 12, LICA 38, LICA 44, LICA 97, LICA 99, KR OPPR und KR OPP2, sowie die von DuPont unter dem Handelsnamen Tyzor^{®} erhältlichen Typen ET, TPT, NPT, BTM, AA, AA-75, AA-95, AA-105, TE, ETAM und OGT; Silane, insbesondere Aminosilane, wie beispielsweise 3-Aminopropyltrimethoxysilan, N-(2-Aminoethyl)-3-aminopropyl-trimethoxysilan, Bis-[3-(trimethoxysilyl)-propyl]-amin, 3-Aminopropyl-dimethoxymethylsilan, 3-Amino-2-methylpropyl-trimethoxysilan, N-(2-Aminoethyl)-3-aminopropyl-dimethoxymethylsilan, 4-Aminobutyl-trimethoxysilan, 4-Aminobutyl-dimethoxymethylsilan, 4-Amino-3-methylbutyl-trimethoxysilan, 4-Amino-3,3-dimethylbutyl-trimethoxysilan, 4-Amino-3,3-dimethylbutyl-dimethoxymethylsilan, [3-(2-Aminoethylamino)-propyl]trimethoxysilan (= 4,7,10-Triazadecyl-trimethoxysilan), 2-Aminoethyl-trimethoxysilan, 2-Aminoethyl-dimethoxymethylsilan, Aminomethyl-trimethoxysilan, Aminomethyl-dimethoxymethylsilan, Aminomethylmethoxydimethylsilan, 7-Amino-4-oxaheptyldimethoxymethylsilan, N-(Methyl)-3-aminopropyltrimethoxysilan, N-(n-butyl)-3-aminopropyltrimethoxysilan; Tris-[3-(trimethoxysilyl)-propyl]-amin, 1,3,5-tris[3-(trimethoxysilyl)propyl]-1,3,5-triazine-2,4,6(1 H,3H,5H)-trion-harnstoff (=Tris(3-(trimethoxysilyl)propyl)isocyanurat) und sowie die ensprechendenen Analoga, bei welchen die Methoxygruppe durch einer Ethoxy- oder Isoproxygruppe ersetzt ist; Mercaptosilane, wie beispielsweise 3-Mercaptopropyltrimethoxysilan oder 3-Mercaptopropyltriethoxysilan, Epoxysilane, wie beispielsweise 3-Glycidyloxypropyltrimethoxysilan, 3-Glycidyloxypropyltriethoxysilan; Ureidoalkylsilane sowie Addukte von Amino- und /oder Mercaptosilanen mit Epoxiden oder Epoxysilanen, beispielsweise mit 3-Glycidyloxypropylsilanen.

Vorteilhaft enthält die beschriebene Beschichtung mindestens einen Haftvermittler in der Form der bereits erwähnten Titanate oder Silane.

Vorteilhaft enthält die beschriebene Beschichtung mindestens einen der bereits erwähnten Katalysatoren.

Die beschriebene Beschichtung wird üblicherweise mittels Pinsel, Filz, Tuch, Schwamm oder einer Spritzpistole auf das Substrat aufgetragen. Dieser Auftrag kann manuell oder automatisch, insbesondere mittels Roboter, erfolgen.

Die beschriebene Beschichtung reagiert bei Kontakt mit Wasser, beispielsweise in Form von Luftfeuchtigkeit, in der bereits beschriebenen Weise, wobei weitere mit Wasser reaktive Komponenten, wie beispielsweise Titanat- oder Silangruppen-haltige Verbindungen, ebenfalls mit Wasser reagieren. Zudem beginnen nach erfolgtem Auftrag der Beschichtung gegebenenfalls darin enthaltene flüchtige Lösemittel zu verdunsten. In der Folge bildet sich auf dem Substrat ein fester, gut haftender Film. Dessen Schichtdicke liegt vorteilhafterweise bei ungefähr 5 - 100 µm, insbesondere 10-50 µm.

Ein weiterer Aspekt der vorliegenden Erfindung betrifft ein Verfahren zum Verkleben von einem Substrat **S1** mit einem Substrat **S2,** welches die Schritte umfasst:
i) Applikation einer der vorgängig beschriebenen Zusammensetzung auf ein Substrat **S1;**
ii) Kontaktieren der applizierten Zusammensetzung mit einem Substrat **S2** innerhalb der Offenzeit der Zusammensetzung;
oder
i') Applikation einer der vorgängig beschriebenen Zusammensetzung auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

Ein weiterer Aspekt der vorliegenden Erfindung betrifft ein Verfahren zum Abdichten. Dieses umfasst den Schritt:
i") Applikation einer vorgängig beschriebenen Zusammensetzung zwischen ein Substrat **S1** und ein Substrat **S2,** so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht. Üblicherweise wird der Dichtstoff in eine sogenannte Fuge eingepresst.

Ein weiterer Aspekt der vorliegenden Erfindung betrifft ein Verfahren zum Beschichten eines Substrates **S1.** Dieses umfasst den Schritt:
i"') Applikation einer vorgängig beschriebenen Zusammensetzung auf ein Substrat **S1** innerhalb der Offenzeit der Zusammensetzung.

In diesen drei Verfahren sind geeignete Substrate **S1** und/oder **S2** beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Leder, Stoffe, Papier, Holz, mit Harz gebundene Holzwerkstoffe, Harz-Texil-Compositewerkstoffe, Kunststoffe wie PVC, Polycarbonate, PMMA, Polyester, Epoxidharze, Polyurethane (PUR); beschichtete Substrate wie pulverbeschichtete Metalle oder Legierungen; sowie Farben und Lacke, insbesondere Automobillacke.

Als Kunststoffe eignen sich insbesondere Polyvinylchlorid (PVC), Acrylonitril-Butadien-Styrol-Copolymere (ABS), SMC (Sheet Moulding Composites), Polycarbonat (PC), Polyamid (PA), Polyester, PMMA, Polyester, Epoxidharze, Polyurethane (PUR), Polyoxymethylen (POM), Polyolefine (PO), insbesondere mittels Plasma, Corona oder Flammen oberflächenbehandeltes Polyethylen (PE) oder Polypropylen (PP), Ethylen/Propylen-Copolymere (EPM) und Ethylen/Propylen-Dien-Terpolymere (EPDM).

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Die Applikation der Zusammensetzung kann in einem breiten Temperaturspektrum erfolgen. Beispielsweise kann die Zusammensetzung bei Raumtemperatur appliziert werden, wie es für einen elastischen Klebstoff oder einen Dichtstoff typisch ist. Die Zusammensetzung kann aber auch bei tieferen wie auch bei höheren Temperaturen appliziert werden. Letzteres ist insbesondere dann vorteilhaft, wenn die Zusammensetzung hochviskose oder schmelzbare Komponenten enthält, wie sie in Schmelzklebstoffen, beispielsweise Warmschmelzklebstoffen (Warm-Melt) oder Heissschmelzklebstoffen (Hot-Melt) typischerweise vorhanden sind. Die Applikationstemperaturen liegen für Warm-Melts beispielsweise im Bereich von 40 bis 80 °C, bei Hot-Melts im Bereich von 85 bis 200 °C.

Somit wird im Falle eines Warm-Melts die Zusammensetzung vor der Applikation auf eine Temperatur 40 °C bis 85 °C, insbesondere von 60 °C bis 80 °C, aufgeheizt und wird insbesondere bei dieser Temperatur in Schritt i) oder i') oder i") oder i"') des oben beschriebenen Verfahrens appliziert. Im Falle eines Hot-Melts wird die Zusammensetzung vor der Applikation auf eine Temperatur von 85 °C bis 200 °C, insbesondere von 100°C bis 180°C, bevorzugt von 120°C bis 160°C, aufgeheizt und wird insbesondere bei dieser Temperatur in Schritt i) oder i') oder i") oder i"') des oben beschriebenen Verfahrens appliziert.

An den Schritt iii) schliesst sich typischerweise ein Schritt iv) des chemischen Vernetzens der Zusammensetzung mit Feuchtigkeit an. Der Fachmann versteht, dass die Vernetzungsreaktion, in Abhängigkeit von Faktoren wie der verwendeten Zusammensetzung, der Substrate, der Temperatur, der Umgebungsfeuchtigkeit und der Klebegeometrie, bereits während der Verklebung beginnen kann. Der Hauptteil der Vernetzung findet im Allgemeinen jedoch nach der Verklebung statt.

Aus diesen beschrieben Verfahren zum Verkleben, Abdichten bzw. Beschichten entsteht ein Artikel.

Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

### Beispiele

### Beschreibung der Messmethoden

**Infrarotspektren** wurden auf einem FT-IR Gerät 1600 von Perkin-Elmer (horizontale ATR-Messeinheit mit ZnSe-Kristall) gemessen, wobei die Substanzen unverdünnt als Film aufgetragen wurden. Die Absorptionsbanden sind in Wellenzahlen (cm⁻¹) angegeben (Messfenster: 4000-650 cm⁻¹).

**¹H-NMR-Spektren** wurden auf einem Spektrometer des Typs Bruker DPX-300 bei 300.13 MHz gemessen; die chemischen Verschiebungen δ sind angegeben in ppm relativ zu Tetramethylsilan (TMS), Kopplungskonstanten *J* sind angegeben in Hz. Echte und Pseudo-Kopplungsmuster wurden nicht unterschieden.

Die **Viskosität** wurde bei der angegebenen Temperatur auf einem thermostatisierten Kegel-Platten-Viskosimeter Physica UM (Kegeldurchmesser 20 mm, Kegelwinkel 1°, Kegelspitze-Platten-Abstand 0.1 mm, Schergeschwindigkeit 10 bis 1000 s⁻¹) gemessen.

Der **Amingehalt** der hergestellten Dialdimine, das heisst der Gehalt an geschützten Aminogruppen in Form von Aldiminogruppen, wurde titrimetrisch bestimmt (mit 0.1N HClO₄ in Eisessig, gegen Kristallviolett) und ist stets angegeben in mmol N/g.

Der **Gehalt an monomeren Diisocyanaten** wurde bestimmt mittels HPLC (Detektion über Photodiodenarray; 0.04 M Natriumacetat / Acetonitril als mobile Phasen) und ist angegeben in Gewichts-% bezogen auf die gesamte Zusammensetzung.

Die **Zugfestigkeit,** die **Bruchdehnung** und das **E-Modul** wurden in Anlehnung an DIN 53504 bestimmt, an Hanteln mit einer Dicke von 1 mm und einer Länge von 75 mm (Steglänge 30 mm, Stegbreite 4 mm). Zur Herstellung der Hanteln wurde ein Klebstofffilm von 1 mm Dicke zubereitet (Applikationstemperatur des Klebstoffs 130 °C), aus welchem die Hanteln ausgestanzt und anschliessend während der angegebenen Zeit bei 23 °C und 50% relativer Luftfeuchtigkeit gelagert wurden.

### a) Herstellung von Aldiminen der Formel (I)

### Beispiel 1: Aldimin A-1

In einem Rundkolben wurden unter Stickstoffatmosphäre 16.74 g 3-Acetoxy-2,2-dimethyl-propanal vorgelegt. Unter kräftigem Rühren wurden aus einem Eintropftrichter 12.00 g 2-(2-Aminoethoxy)-ethanol (Diglycolamine^{®} Agent; Huntsman) langsam zugegeben, wobei sich die Mischung erwärmte. Danach wurden die flüchtigen Bestandteile im Vakuum entfernt (10 mbar, 100 °C). Ausbeute: 26.5 g einer farblosen, klaren Flüssigkeit mit einem Amingehalt von 4.25 mmol N/g und einer Viskosität von 10 mPa·s bei 20 °C.
IR: 3415br (O-H), 2962, 2928, 2914, 2902, 2888sh, 2866, 2849sh, 2722br, 1736 (C=O), 1666 (C=N), 1470, 1458sh, 1439sh, 1395, 1374, 1234, 1126, 1038, 1009, 988, 931, 894, 847, 814, 793sh.
¹H-NMR (CDCl₃, 300 K): δ 7.59 (*t*, 1 H, *J ≈* 1.3, CH=N), 4.03 (s, 2 H, CH₂O), 3.71 (m, 4 H, HOCH₂C*H*₂OC*H*₂CH₂N), 3.58 (m, 4 H, HOC*H*₂CH₂OCH₂C*H*₂N), 3.06 (s, 1 H, OH), 2.06 (s, 3 H, CH₃CO), 1.11 (s, 6 H, C(CH₃)₂).

### Beispiel 2: Aldimin A-2

In einem Rundkolben wurden unter Stickstoffatmosphäre 14.39 g 3-Acetoxy-2,2-dimethyl-propanal vorgelegt. Unter kräftigem Rühren wurden portionsweise 10.00 g festes 5-Amino-1-pentanol zugegeben, wobei letzteres in Lösung ging und sich die Mischung erwärmte. Danach wurden die flüchtigen Bestandteile im Vakuum entfernt (10 mbar, 100 °C). Ausbeute: 22.4 g einer farblosen, klaren Flüssigkeit mit einem Amingehalt von 4.28 mmol N/g und einer Viskosität von 50 mPa·s bei 20 °C.
IR: 3380br (O-H), 2932, 2860, 2723br, 1738 (C=O), 1666 (C=N), 1470, 1458, 1437sh, 1395, 1374, 1238, 1069sh, 1038, 1012sh, 988, 928, 891, 846, 775,734.
¹H-NMR (CDCl₃, 300 K): δ 7.52 (*t*, 1 H, *J ≈* 1.2, CH=N), 4.02 (s, 2 H, CH₂O), 3.63 (*t*, 2 H, *J ≈* 6.5, HOC*H*₂CH₂CH₂), 3.39 (*t*×*d*, 2 H, *J ≈* 6.9 / 1.2, CH=NC*H*₂CH₂), 2.11 (s, 1 H, OH), 2.05 (s, 3 H, CH₃CO), 1.60 (m, 4 H, HOCH₂C*H*₂CH₂ und CH=NCH₂C*H*₂), 1.36 (*m*, 2 H, HOCH₂CH₂C*H*₂), 1.11 (s, 6 H, C(CH₃)₂).

### Beispiel 3: Aldimin A-3

In einem Rundkolben wurden unter Stickstoffatmosphäre 15.94 g 3-Acetoxy-2,2-dimethyl-propanal vorgelegt. Unter kräftigem Rühren wurden portionsweise 12.00 g bei 60 °C verflüssigtes Bis-hexamethylentriamin (BHMT-HP; Invista) zupipettiert, wobei sich die Mischung erwärmte. Danach wurden die flüchtigen Bestandteile im Vakuum entfernt (10 mbar, 100 °C). Ausbeute: 26.1 g einer farblosen, klaren Flüssigkeit mit einem Amingehalt von 6.24 mmol N/g und einer Viskosität von 60 mPa·s bei 20 °C.
IR: 3313 (N-H), 2963sh, 2928, 2852, 2826, 1738 (C=O), 1668 (C=N), 1468, 1437sh, 1394, 1374, 1339sh, 1234, 1128, 1038, 1011sh, 986, 928, 844,730,668.
¹H-NMR (CDCl₃, 300 K): δ 7.51 (*t*, 2 H, *J* ≈ 1.2, CH=N), 4.02 (s, 4 H, CH₂O), 3.36 (*t*×d*,* 4 H, *J* ≈ 6.9 / 1.2, CH=NC*H*₂CH₂), 2.58 (*t,* 4 H, *J* ≈ 7.2, NHC*H*₂CH₂CH₂), 2.04 (s, 6 H, CH₃CO), 1.63-1.43 (m, 9 H, CH=NCH₂C*H*₂CH₂ und N*H*CH₂C*H*₂CH₂), 1.30 (*m*, 8 H, CH=NCH₂CH₂C*H*₂ und NHCH₂CH₂C*H*₂), 1.10 (s, 12 H, C(CH₃)₂).

### b) Herstellung von Polyurethanpolymeren

### Polyurethanpolymer PUP-1

780 g Polyol Dynacoll^{®} 7360 (Degussa; kristallines Polyester-Diol, OH-Zahl 32 mg KOH/g, Säurezahl ca. 2 mg KOH/g) und 120 g 4,4'-Methylen-diphenyldiisocyanat (MDI; Desmodur^{®} 44 MC L, Bayer) wurden nach bekanntem Verfahren bei 80 °C zu einem NCO-terminierten Polyurethanpolymeren umgesetzt. Das bei Raumtemperatur feste Reaktionsprodukt hatte einen titrimetrisch bestimmten Gehalt an freien Isocyanatgruppen von 1.97 Gewichts-%.

### Polyurethanpolymer PUP-2

435 g Polyol Dynacoll^{®} 7360 (Degussa; kristallines Polyester-Diol, OH-Zahl 32 mg KOH/g, Säurezahl ca. 2 mg KOH/g) und 65 g Isophorondiisocyanat (IPDI; Vestanat^{®} IPDI, Degussa) wurden nach bekanntem Verfahren bei 80 °C zu einem NCO-terminierten Polyurethanpolymeren umgesetzt. Das bei Raumtemperatur feste Reaktionsprodukt hatte einen titrimetrisch bestimmten Gehalt an freien Isocyanatgruppen von 2.25 Gewichts-%.

### c) Herstellung von Heissschmelzklebstoffen

### Beispiele 4 bis 6 und Vergleichsbeispiel 7

Für jedes Beispiel wurden die jeweiligen Bestandteile gemäss Tabelle 1 auf 100 °C erwärmt und unter Stickstoffatmosphäre in den angegebenen Gewichtsteilen in einen Polypropylenbecher mit Schraubverschluss eingewogen und mittels eines Zentrifugalmischers (SpeedMixer™ DAC 150, FlackTek Inc.; 1 min. bei 3000 U/min) gemischt. Die so erhaltene dünnflüssige Mischung wurde unmittelbar danach in eine innenlackierte Aluminiumtube abgefüllt, diese luftdicht verschlossen und während 1 Stunde bei 100 °C gelagert.

**Tabelle 1: Zusammensetzung der Heissschmelzklebstoffe der Beispiele 4 bis 6 und des Vergleichsbeispiels 7.**

| Beispiel | **4** | **5** | **6** | **7** **(Vgl.)** |
|---|---|---|---|---|
| PUR-Polymer ***PUP-1*** | 50.0 | 50.0 | 50.0 | 50.0 |
| Aldimin | ***A-1***, 2.49 | ***A-2***, 2.47 | ***A-3***, 3.38 | - |

In den Beispielen 4 bis 6 beträgt das Verhältnis zwischen den Isocyanatgruppen des Polyurethanpolymers ***PUP-1*** und der Summe der gegenüber Isocyanatgruppen reaktiven Gruppen (Hydroxylgruppen, sekundäre Aminogruppen und Aldiminogruppen) der Aldimine 1.0 / 0.9.

Die so hergestellten Heissschmelzklebstoffe der Beispiele 4 bis 6 und des Vergleichsbeispiels 7 wurden auf Viskosität und Gehalt an monomerem 4,4'-Methylendiphenyldiisocyanat (4,4'-MDI) geprüft. Die Zugfestigkeit, Bruchdehnung und das E-Modul wurde am während 7 Tagen gelagerten Klebstofffilm gemessen. Die Ergebnisse sind in der Tabelle 2 dargestellt.

**Tabelle 2: Eigenschaften der Heissschmelzklebstoffe der Beispiele 4 bis 6 und des Vergleichsbeispiels 7.**

| Beispiel | **4** | **5** | **6** | **7** **(Vgl.)** |
|---|---|---|---|---|
| Gehalt an monomeren 4,4'-MDI [(Gewichts-%)] | 0.21 | 0.34 | 0.43 | 1.59 |
| Viskosität bei 130 °C [Pa·s] | 6.2 | 6.4 | 9.0 | 7.0 |
| Zugfestigkeit [MPa] | 18.1 | 21.1 | 26.0 | 18.7 |
| Bruchdehnung [%] | 730 | 650 | 360 | 510 |
| E-Modul bei 0.5-5.0% [MPa] | 195 | 235 | 220 | 315 |

### Beispiel 8 und Vergleichsbeispiel 9

Für jedes Beispiel wurden die jeweiligen Bestandteile gemäss Tabelle 3 auf 100 °C erwärmt und unter Stickstoffatmosphäre in den angegebenen Gewichtsteilen in einen Polypropylenbecher mit Schraubverschluss eingewogen und mittels eines Zentrifugalmischers (SpeedMixer™ DAC 150, FlackTek Inc.; 1 min. bei 3000 U/min) gemischt. Die so erhaltene dünnflüssige Mischung wurde unmittelbar danach in eine innenlackierte Aluminiumtube abgefüllt, diese luftdicht verschlossen und während 1 Stunde bei 100 °C gelagert.

**Tabelle 3: Zusammensetzung der Heissschmelzklebstoffe des Beispiels 8 und des Vergleichsbeispiels 9.**

| Beispiel | **8** | **9** **(Vergleich)** |
|---|---|---|
| PUR-Polymer ***PUP-1*** | 50.0 | 50.0 |
| Aldimin ***A-1*** | 2.84 | - |

Im Beispiel 8 beträgt das Verhältnis zwischen den Isocyanatgruppen des Polyurethanpolymers ***PUP-2*** und der Summe der Hydroxylgruppen und Aldiminogruppen des Aldimins ***A-1*** 1.0 / 0.9.

Die so hergestellten Heissschmelzklebstoffe des Beispiels 8 und des Vergleichsbeispiels 9 wurden auf Viskosität und Gehalt an monomerem Isophorondiisocyanat (IPDI; Summe aus *cis-* und trans-Isomeren) geprüft. Die Zugfestigkeit, Bruchdehnung und das E-Modul wurde am während 3 Wochen gelagerten Klebstofffilm gemessen. Die Ergebnisse sind in der Tabelle 4 dargestellt.

**Tabelle 4: Eigenschaften der Heissschmelzklebstoffe des Beispiels 8 und des Vergleichsbeispiels 9.**

| Beispiel | **8** | **9** **(Vergleich)** |
|---|---|---|
| Gehalt an monomeren IPDI [(Gewichts-%)] | 0.21 | 2.32 |
| Viskosität bei 130 °C [Pa·s] | 2.4 | 2.1 |
| Zugfestigkeit [MPa] | 17.7 | 18.2 |
| Bruchdehnung [%] | 800 | 9 |
| E-Modul bei 0.5-5.0% [MPa] | 200 | 305 |

## Patentansprüche

1. Aldimin der Formel (I) 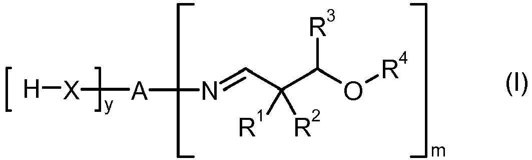 wobei
R¹ und R² entweder
unabhängig voneinander jeweils für einen einwertigen Kohlenwasserstoffrest mit 1 bis 12 C-Atomen stehen,
oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder für eine Alkyl- oder Arylalkylgruppe, insbesondere mit 1 bis 12 C-Atomen, steht;
R⁴ entweder
für einen Kohlenwasserstoffrest mit 1 bis 30 C-Atomen, der gegebenenfalls Ethersauerstoffatome aufweist, steht,
oder für einen Rest 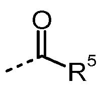 steht, wobei R⁵ für ein Wasserstoffatom oder für einen Kohlenwasserstoffrest mit 1 bis 5 C-Atomen steht;
A entweder
für einen (m+y)-wertigen, gegebenenfalls Heteroatome aufweisenden, Kohlenwasserstoffrest mit 2 bis 20 C-Atomen steht, oder, falls y für 1 und X für N-R⁷ steht, zusammen mit R⁷ für einen (m+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht;
X für O oder S oder N-R⁶ oder N-R⁷ steht,
wobei R⁶
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (II) steht, 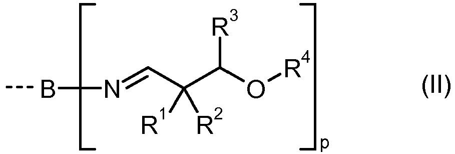 wobei
p für 0 oder für eine ganze Zahl von 1 bis 10'000 steht, und
B für einen (p+1)-wertigen Kohlenwasserstoffrest, welcher gegebenenfalls Heteroatome, insbesondere in Form von Ether-Sauerstoff oder tertiärem Amin-Stickstoff, und gegebenenfalls aktiven Wasserstoff in Form von Hydroxylgruppen, sekundären Aminogruppen oder Mercaptogruppen enthält, steht, und
R⁷ zusammen mit A für einen (m+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht; und
m für 1 oder 2 oder 3 oder 4 steht, und
y für 1 oder 2 oder 3 oder 4 steht.

2. Aldimin gemäss Anspruch 1, **dadurch gekennzeichnet, dass** es erhalten wird aus der Umsetzung von mindestens einem Amin **B1** der Formel (III) mit mindestens einem Aldehyd **ALD** der Formel (IV), 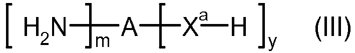 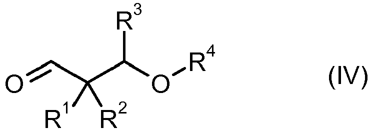 wobei
X^{a} für O oder S oder N-R^{6a} oder N-R⁷ steht,
wobei R^{6a} entweder
für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (III a) steht.

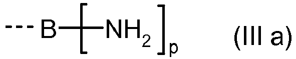

3. Aldiminogruppen-haltige Verbindung **AV**, **dadurch gekennzeichnet, dass** diese durch die Umsetzung eines Aldimins der Formel (I) gemäss einem der Ansprüche 1 bis 2 mit einer Verbindung **D,** die mehr als eine Reaktivgruppe trägt, welche mit einer Gruppe HX Additionsreaktionen eingehen kann, erhalten wird.

4. Aldiminogruppen-haltige Verbindung gemäss Anspruch 3, **dadurch gekennzeichnet, dass** diese eine Aldiminogruppen-haltige Verbindung **AV1** der Formel (VI) ist, 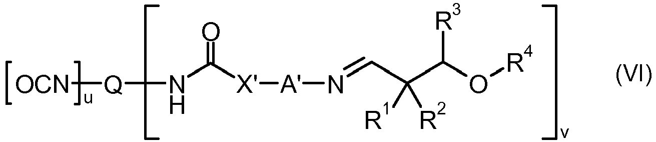 wobei
u für 0 oder 1 oder 2 oder 3 oder 4 oder 5 steht,
v für 1 oder 2 oder 3 oder 4 oder 5 oder 6 steht,
mit der Massgabe, dass (u+v) für 2 oder 3 oder 4 oder 5 oder 6 steht;
Q für den Rest eines (u+v) Isocyanatgruppen aufweisenden Polyisocyanates nach Entfernung aller Isocyanatgruppen steht;
A' entweder
für einen zweiwertigen, gegebenenfalls Heteroatome aufweisenden, Kohlenwasserstoffrest mit 2 bis 20 C-Atomen steht,
oder zusammen mit R^{7'} für einen dreiwertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht; und
X' für O oder S oder N-R^{6'} oder N-R^{7'} steht,
wobei R^{6'}
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (II') steht, 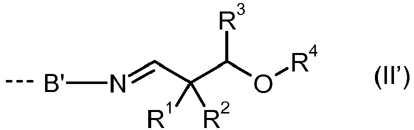 wobei B' für einen zweiwertigen, gegebenenfalls Heteroatome aufweisenden, Kohlenwasserstoffrest mit 2 bis 20 C-Atomen steht, und
R^{7'} zusammen mit A' für einen dreiwertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht.

5. Aldiminogruppen-haltige Verbindung **AV1** gemäss Anspruch 4, **dadurch gekennzeichnet, dass** Q für ein (u+v) endständige Isocyanatgruppen aufweisendes Polyurethanpolymer **PUP** nach Entfernung aller Isocyanatgruppen steht, welches insbesondere erhältlich ist aus der Umsetzung von mindestens einem Polyol mit mindestens einem Polyisocyanat, vorzugsweise aus mindestens einem Diol und mindestens einem Diisocyanat.

6. Aldiminogruppen-haltige Verbindung **AV1** gemäss Anspruch 4, **dadurch gekennzeichnet, dass** Q für ein (u+v) endständige Isocyanatgruppen aufweisendes Polyisocyanat **PI** in der Form eines aliphatischen, cycloaliphatischen oder aromatischen Diisocyanates, oder eines niedrigmolekularen Oligomeren dieser Diisocyanate, oder eines Derivates dieser Diisocyanate, nach Entfernung aller Isocyanatgruppen steht.

7. Zusammensetzung enthaltend ein Aldimin gemäss einem der Ansprüche 1 bis 2 oder eine Aldiminogruppen-haltige Verbindung gemäss einem der Ansprüche 3 bis 6.

8. Zusammensetzung gemäss Anspruch 7, **dadurch gekennzeichnet, dass** die Zusammensetzung
a) mindestens eine Aldiminogruppen-haltige Verbindung **AV1** gemäss Anspruch 4 bis 6 und
b) gegebenenfalls mindestens ein Polyisocyanat **P** enthält,
mit der Massgabe, dass das Verhältnis der Anzahl Aldiminogruppen zu der Anzahl Isocyanatgruppen in der Zusammensetzung höchstens 1 beträgt.

9. Zusammensetzung gemäss Anspruch 8, **dadurch gekennzeichnet, dass** die Zusammensetzung einen Gehalt an monomeren Diisocyanaten von ≤ 1 Gewichts-%, insbesondere von ≤ 0.5 Gewichts-%, bezogen auf die feuchtigkeitsreaktiven Bestandteile der Zusammensetzung, aufweist.

10. Ausgehärtete Zusammensetzung, welche durch die Reaktion einer Zusammensetzung gemäss einem der Ansprüche 7 bis 9 mit Wasser, insbesondere in Form von Luftfeuchtigkeit, erhalten wird.

11. Verwendung einer Zusammensetzung gemäss einem der Ansprüche 7 bis 9 als Klebstoff, Dichtstoff, Vergussmasse oder Beschichtung.

12. Verfahren zum Verkleben von einem Substrat **S1** mit einem Substrat **S2,** umfassend die Schritte
i) Applikation einer Zusammensetzung gemäss einem der Ansprüche 7 bis 9 auf ein Substrat **S1;**
ii) Kontaktieren der applizierten Zusammensetzung mit einem Substrat S2 innerhalb der Offenzeit der Zusammensetzung;
oder
i') Applikation einer Zusammensetzung gemäss einem der Ansprüche 7 bis 9 auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

13. Verfahren zum Abdichten, umfassend den Schritt
i") Applikation einer Zusammensetzung gemäss einem der Ansprüche 7 bis 9 zwischen ein Substrat **S1** und ein Substrat **S2,** so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht.

14. Verfahren zum Beschichten eines Substrates **S1,** umfassend den Schritt
i"') Applikation einer Zusammensetzung gemäss einem der Ansprüche 7 bis 9 auf ein Substrat **S1** innerhalb der Offenzeit der Zusammensetzung.

15. Artikel, welcher nach einem Verfahren gemäss einem der Ansprüche 12 bis 14 verklebt, abgedichtet oder beschichtet wurde.

16. Verfahren zur Verminderung des Gehalts an monomeren Diisocyanaten in Isocyanatgruppen aufweisenden Polyurethanpolymeren oder in oligomeren Polyisocyanaten, oder in Zusammensetzungen, welche Isocyanatgruppen aufweisende Polyurethanpolymere oder oligomere Polyisocyanate enthalten, in dem diese mit mindestens einem Aldimin der Formel (I) gemäss Anspruch 1 oder 2 umgesetzt werden.

## Claims

1. Aldimine of the formula (I) 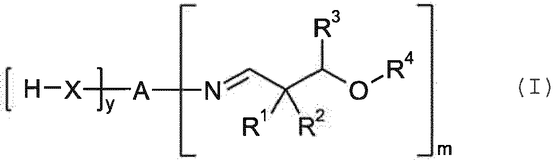 where
R¹ and R² are either
each independently a monovalent hydrocarbon radical having 1 to 12 carbon atoms,
or together a divalent hydrocarbon radical which has 4 to 12 carbon atoms and is part of an optionally substituted carbocyclic ring having 5 to 8, preferably 6, carbon atoms;
R³ is a hydrogen atom or an alkyl or arylalkyl group, especially having 1 to 12 carbon atoms;
R⁴ is either
a hydrocarbon radical which has 1 to 30 carbon atoms and optionally has ether oxygen atoms,
or a 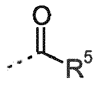 radical where R⁵ is a hydrogen atom or is a hydrocarbon radical having 1 to 5 carbon atoms;
A is either
an (m+y)-valent hydrocarbon radical which has 2 to 20 carbon atoms and optionally has heteroatoms,
or, if y is 1 and X is N-R⁷, together with R⁷, an (m+2)-valent hydrocarbon radical which has 3 to 20 carbon atoms and optionally contains at least one heteroatom, especially in the form of ether oxygen or tertiary amine nitrogen;
X is O or S or N-R⁶ or N-R⁷,
where R⁶
is either a monovalent hydrocarbon radical which has 1 to 20 carbon atoms and optionally has at least one carboxylic ester, nitrile, nitro, phosphonic ester, sulfone or sulfonic ester group,
or a substituent of the formula (II) 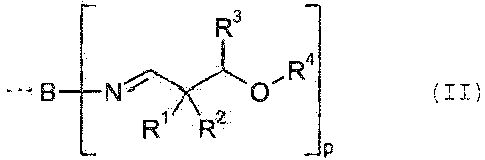 where
p is 0 or an integer from 1 to 10 000, and
B is a (p+1)-valent hydrocarbon radical which optionally contains heteroatoms, especially in the form of ether oxygen or tertiary amine nitrogen, and optionally active hydrogen in the form of hydroxyl groups, secondary amino groups or mercapto groups, and
R⁷ together with A is an (m+2)-valent hydrocarbon radical which has 3 to 20 carbon atoms and optionally contains at least one heteroatom, especially in the form of ether oxygen or tertiary amine nitrogen; and
m is 1 or 2 or 3 or 4, and
y is 1 or 2 or 3 or 4.

2. Aldimine according to Claim 1, **characterized in that** it is obtained from the reaction of at least one amine **B1** of the formula (III) with at least one aldehyde **ALD** of the formula (IV) 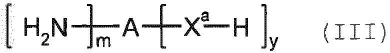 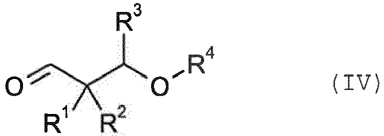 where
X^{a} is 0 or S or N-R^{6a} or N-R⁷,
where R^{6a} is either
a monovalent hydrocarbon radical which has 1 to 20 carbon atoms and optionally has at least one carboxylic ester, nitrile, nitro, phosphonic ester, sulfone or sulfonic ester group,
or a substituent of the formula (III a)

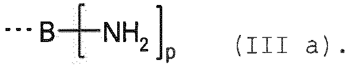

3. Compound **AV** containing aldimino groups, **characterized in that** it is obtained by the reaction of an aldimine of the formula (I) according to either one of Claims 1 and 2 with a compound **D** which bears more than one reactive group which can enter into addition reactions with an HX group.

4. Compound containing aldimino groups according to Claim 3, **characterized in that** it is a compound **AV1** which contains aldimino groups and is of the formula (VI) 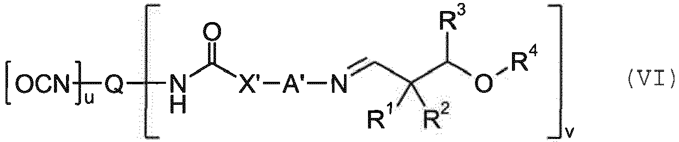 where
u is 0 or 1 or 2 or 3 or 4 or 5,
v is 1 or 2 or 3 or 4 or 5 or 6,
with the proviso that (u+v) is 2 or 3 or 4 or 5 or 6;
Q is the radical of a polyisocyanate having (u+v) isocyanate groups after removal of all isocyanate groups;
A' is either
a divalent hydrocarbon radical which has 2 to 20 carbon atoms and optionally has heteroatoms, or, together with R^{7'}, a trivalent hydrocarbon radical which has 3 to 20 carbon atoms and optionally contains at least one heteroatom, especially in the form of ether oxygen or tertiary amine nitrogen; and
X' is O or S or N-R^{6'} or N-R^{7'},
where R^{6'}
is either a monovalent hydrocarbon radical which has 1 to 20 carbon atoms and optionally has at least one carboxylic ester, nitrile, nitro, phosphonic ester, sulfone or sulfonic ester group,
or a substituent of the formula (II') 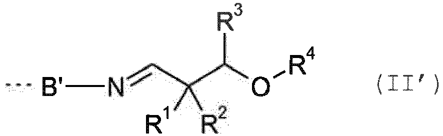 where B' is a divalent hydrocarbon radical which has 2 to 20 carbon atoms and optionally has heteroatoms, and
R^{7'} together with A' is a trivalent hydrocarbon radical which has 3 to 20 carbon atoms and optionally contains at least one heteroatom, especially in the form of ether oxygen or tertiary amine nitrogen.

5. Compound **AV1** containing aldimino groups according to Claim 4, **characterized in that** Q is a polyurethane polymer **PUP** having (u+v) terminal isocyanate groups after removal of all isocyanate groups, which is especially obtainable from the reaction of at least one polyol with at least one polyisocyanate, preferably from at least one diol and at least one diisocyanate.

6. Compound **AV1** containing aldimino groups according to Claim 4, **characterized in that** Q is a polyisocyanate **PI** having (u+v) terminal isocyanate groups in the form of an aliphatic, cycloaliphatic or aromatic diisocyanate, or of a low molecular weight oligomer of these diisocyanates, or of a derivative of these diisocyanates, after removal of all isocyanate groups.

7. Composition comprising an aldimine according to either one of Claims 1 and 2 or a compound containing aldimino groups according to any one of Claims 3 to 6.

8. Composition according to Claim 7, **characterized in that** the composition comprises
a) at least one compound **AV1** containing aldimino groups according to Claims 4 to 6 and
b) optionally at least one polyisocyanate **P,**
with the proviso that the ratio of the number of aldimino groups to the number of isocyanate groups in the composition is at most 1.

9. Composition according to Claim 8, **characterized in that** the composition has a content of monomer diisocyanates of ≤ 1% by weight, especially of ≤ 0.5% by weight, based on the moisture-reactive constituents of the composition.

10. Cured composition obtained by the reaction of a composition according to any one of Claims 7 to 9 with water, especially in the form of air humidity.

11. Use of a composition according to any one of Claims 7 to 9 as an adhesive, sealant, potting composition or coating.

12. Process for adhesive bonding a substrate **S1** to a substrate **S2,** comprising the steps of
i) applying a composition according to any one of Claims 7 to 9 to a substrate **S1;**
ii) contacting the composition applied to a substrate **S2** within the open time of the composition;
or
i') applying a composition according to any one of Claims 7 to 9 to a substrate **S1** and to a substrate **S2;**
ii') contacting the composition applied with one another within the open time of the composition;
the substrate **S2** consisting of the same material as, or a different material than, the substrate **S1.**

13. Process for sealing, comprising the step of
i") applying a composition according to any one of Claims 7 to 9 between a substrate **S1** and a substrate **S2,** such that the composition is in contact with the substrate **S1** and the substrate **S2;**
the substrate **S2** consisting of the same material as, or a different material than, the substrate **S1.**

14. Process for coating a substrate **S1,** comprising the step of
i'") applying a composition according to any one of Claims 7 to 9 to a substrate **S1** within the open time of the composition.

15. Article which has been adhesive bonded, sealed or coated by a process according to any one of Claims 12 to 14.

16. Process for reducing the content of monomeric diisocyanates in polyurethane polymers having isocyanate groups, or in oligomeric polyisocyanates, or in compositions which contain polyurethane polymers having isocyanate groups or oligomeric polyisocyanates, by reacting them with at least one aldimine of the formula (I) according to Claims 1 or 2.

## Revendications

1. Aldimine de formule (I) 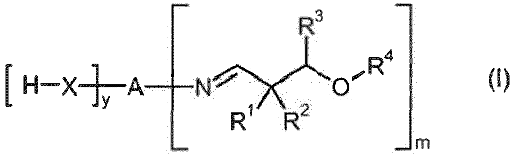 dans laquelle
R¹ et R²
représentent chacun indépendamment l'un de l'autre un radical hydrocarboné monovalent de 1 à 12 atomes C,
ou représentent ensemble un radical hydrocarboné bivalent de 4 à 12 atomes C, qui fait partie d'un cycle carbocyclique éventuellement substitué de 5 à 8, de préférence 6, atomes C ;
R³ représente un atome d'hydrogène ou un groupe alkyle ou arylalkyle, notamment de 1 à 12 atomes C ;
R⁴ représente un radical hydrocarboné de 1 à 30 atomes C, qui comprend éventuellement des atomes d'oxygène éthérés,
ou représente un radical 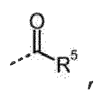 R⁵ représentant un atome d'hydrogène ou un radical hydrocarboné de 1 à 5 atomes C ;
A représente un radical hydrocarboné (m+y)-valent, comprenant éventuellement des hétéroatomes, de 2 à 20 atomes C,
ou, si y représente 1 et X représente N-R⁷, représente conjointement avec R⁷ un radical hydrocarboné (m+2)-valent de 3 à 20 atomes C, qui contient éventuellement au moins un hétéroatome, notamment sous la forme d'un oxygène éthéré ou d'un azote d'amine tertiaire ;
X représente O ou S ou N-R⁶ ou N-R⁷,
R⁶ représentant un radical hydrocarboné monovalent de 1 à 20 atomes C, qui comprend éventuellement au moins un groupe acide carboxylique, ester, nitrile, nitro, ester de l'acide phosphonique, sulfone ou ester de l'acide sulfonique,
ou représentant un substituant de formule (II) 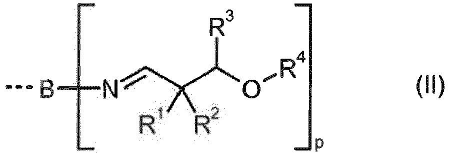 dans laquelle
p représente 0 ou un nombre entier de 1 à 10 000, et
B représente un radical hydrocarboné (p+1)-valent, qui contient éventuellement des hétéroatomes, notamment sous la forme d'un oxygène éthéré ou d'un azote d'amine tertiaire, et éventuellement de l'hydrogène actif sous la forme de groupes hydroxyle, de groupes amino secondaires ou de groupes mercapto, et
R⁷ représentant conjointement avec A un radical hydrocarboné (m+2)-valent de 3 à 20 atomes C, qui contient éventuellement au moins un hétéroatome, notamment sous la forme d'un oxygène éthéré ou d'un azote d'amine tertiaire ; et
m représente 1 ou 2 ou 3 ou 4, et
y représente 1 ou 2 ou 3 ou 4.

2. Aldimine selon la revendication 1, **caractérisée en ce qu'**elle est obtenue par la mise en réaction d'au moins une amine B1 de formule (III) avec au moins un aldéhyde ALD de formule (IV) 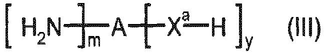 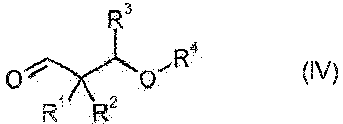 dans lesquelles
X^{a} représente O ou S ou N-R^{6a} ou N-R⁷,
R^{6a} représentant un radical hydrocarboné monovalent de 1 à 20 atomes C, qui comprend éventuellement au moins un groupe acide carboxylique, ester, nitrile, nitro, ester de l'acide phosphonique, sulfone ou ester de l'acide sulfonique,
ou représentant un substituant de formule (IIIa)

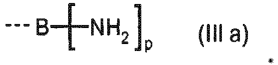

3. Composé contenant des groupes aldimino AV, **caractérisé en ce que** celui-ci est obtenu par mise en réaction d'une aldimine de formule (I) selon l'une quelconque des revendications 1 à 2 avec un composé D qui porte plus d'un groupe réactif pouvant donner lieu à des réactions d'addition avec un groupe HX.

4. Composé contenant des groupes aldimino selon la revendication 3, **caractérisé en ce que** celui-ci est un composé contenant des groupes aldimino AV1 de formule (VI) 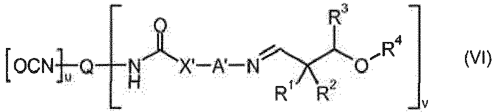 dans laquelle
u représente 0 ou 1 ou 2 ou 3 ou 4 ou 5,
v représente 1 ou 2 ou 3 ou 4 ou 5 ou 6,
à condition que (u+v) représente 2 ou 3 ou 4 ou 5 ou 6 ;
Q représente le radical d'un polyisocyanate comprenant (u+v) groupes isocyanate après l'élimination de tous les groupes isocyanate ;
A' représente un radical hydrocarboné bivalent, comprenant éventuellement des hétéroatomes, de 2 à 20 atomes C,
ou représente conjointement avec R^{7'} un radical hydrocarboné trivalent de 3 à 20 atomes C, qui contient éventuellement au moins un hétéroatome, notamment sous la forme d'un oxygène éthéré ou d'un azote d'amine tertiaire ; et
X' représente O ou S ou N-R^{6'} ou N-R^{7'},
R^{6'} représentant un radical hydrocarboné monovalent de 1 à 20 atomes C, qui comprend éventuellement au moins un groupe acide carboxylique, ester, nitrile, nitro, ester de l'acide phosphonique, sulfone ou ester de l'acide sulfonique,
ou représentant un substituant de formule (II') 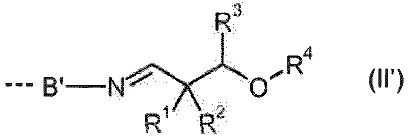 dans laquelle B' représente un radical hydrocarboné bivalent, comprenant éventuellement des hétéroatomes, de 2 à 20 atomes C, et
R^{7'} représentant conjointement avec A' un radical hydrocarboné trivalent de 3 à 20 atomes C, qui contient éventuellement au moins un hétéroatome, notamment sous la forme d'un oxygène éthéré ou d'un azote d'amine tertiaire.

5. Composé contenant des groupes aldimino AV1 selon la revendication 4, **caractérisé en ce que** Q représente un polymère de polyuréthane PUP comprenant (u+v) groupes isocyanate terminaux après l'élimination de tous les groupes isocyanate, qui peut notamment être obtenu par la mise en réaction d'au moins un polyol avec au moins un polyisocyanate, de préférence d'au moins un diol et d'au moins un diisocyanate.

6. Composé contenant des groupes aldimino AV1 selon la revendication 4, **caractérisé en ce que** Q représente un polyisocyanate PI comprenant (u+v) groupes isocyanate terminaux sous la forme d'un diisocyanate aliphatique, cycloaliphatique ou aromatique, ou d'un oligomère de faible poids moléculaire de ces diisocyanates, ou d'un dérivé de ces diisocyanates, après l'élimination de tous les groupes isocyanate.

7. Composition contenant une aldimine selon l'une quelconque des revendications 1 à 2 ou un composé contenant des groupes aldimino selon l'une quelconque des revendications 3 à 6.

8. Composition selon la revendication 7, **caractérisée en ce que** la composition contient
a) au moins un composé contenant des groupes aldimino AV1 selon les revendications 4 à 6, et
b) éventuellement au moins un polyisocyanate P,
à condition que le rapport entre le nombre de groupes aldimino et le nombre de groupes isocyanate dans la composition soit d'au plus 1.

9. Composition selon la revendication 8, **caractérisée en ce que** la composition présente une teneur en diisocyanates monomères ≤ 1 % en poids, notamment ≤ 0,5 % en poids, par rapport aux constituants réactifs à l'humidité de la composition.

10. Composition durcie, qui est obtenue par la réaction d'une composition selon l'une quelconque des revendications 7 à 9 avec de l'eau, notamment sous la forme d'humidité de l'air.

11. Utilisation d'une composition selon l'une quelconque des revendications 7 à 9 en tant qu'adhésif, agent d'étanchéité, pâte de remplissage ou revêtement.

12. Procédé pour coller un substrat S1 avec un substrat S2, comprenant les étapes suivantes :
i) l'application d'une composition selon l'une quelconque des revendications 7 à 9 sur un substrat S1 ;
ii) la mise en contact de la composition appliquée avec un substrat S2 pendant le temps d'ouverture de la composition ;
ou
i') l'application d'une composition selon l'une quelconque des revendications 7 à 9 sur un substrat S1 et sur un substrat S2 ;
ii') la mise en contact des compositions appliquées l'une avec l'autre pendant le temps d'ouverture de la composition ;
le substrat S2 étant constitué d'un matériau identique ou différent du substrat S1.

13. Procédé d'étanchéification, comprenant l'étape suivante :
i") l'application d'une composition selon l'une quelconque des revendications 7 à 9 entre un substrat S1 et un substrat S2, de sorte que la composition soit en contact avec le substrat S1 et le substrat S2,
le substrat S2 étant constitué d'un matériau identique ou différent du substrat S1.

14. Procédé de revêtement d'un substrat S1, comprenant l'étape suivante :
i'") l'application d'une composition selon l'une quelconque des revendications 7 à 9 sur un substrat S1 pendant le temps d'ouverture de la composition.

15. Article, qui a été collé, étanchéifié ou revêtu par un procédé selon l'une quelconque des revendications 12 à 14.

16. Procédé de réduction de la teneur en diisocyanates monomères dans des polymères de polyuréthane comprenant des groupes isocyanate ou dans des polyisocyanates oligomères, ou dans des compositions contenant des polymères de polyuréthane comprenant des groupes isocyanate ou des polyisocyanates oligomères, selon lequel ceux-ci sont mis en réaction avec au moins une aldimine de formule (I) selon la revendication 1 ou 2.
